(19) Europäisches Patentamt

European Patent Office

Office européen des brevets

(11) EP 1 136 933 B1

(12) EUROPEAN PATENT SPECIFICATION

(45) Date of publication and mention
of the grant of the patent:
**09.06.2004 Bulletin 2004/24**

(51) Int Cl.[7]: **G06F 19/00**

(21) Application number: **01105480.6**

(22) Date of filing: **14.03.2001**

(54) **Systems and methods for gene expression analysis**

Systeme und Verfahren zur Genexpressionsanalyse

Systèmes et méthodes pour l'analyse d'expression génique

(84) Designated Contracting States:
**AT BE CH CY DE DK ES FI FR GB GR IE IT LI LU MC NL PT SE TR**

(30) Priority: **15.03.2000 US 189558 P**
**12.12.2000 US 735743**

(43) Date of publication of application:
**26.09.2001 Bulletin 2001/39**

(73) Proprietor: **Affymetrix, Inc.**
**Santa Clara, CA 95051 (US)**

(72) Inventors:
• **Liu, Wei-min**
**Campbell, CA 95008 (US)**
• **Mei, Rui**
**Santa Clara, CA 95051 (US)**
• **Ryder, Thomas B.**
**Los Gatos, CA 95030 (US)**

(74) Representative: **UEXKÜLL & STOLBERG**
**Patentanwälte**
**Beselerstrasse 4**
**22607 Hamburg (DE)**

(56) References cited:
**WO-A-97/27317**

• **LIU W ET AL: "Rank-based algorithms for analysis of microarrays" PROCEEDINGS OF SPIE - MICROARRAYS: OPTICAL TECHNOLOGIES AND INFORMATICS, vol. 4266, June 2001 (2001-06), pages 56-67, XP008007060**
• **"Statistical Algorithms Reference Guide" AFFYMETRIX TECHNICAL NOTES, [Online] 2001, page 1-12 XP002210885 Retrieved from the Internet: <URL:http://www.affymetrix.com/support/technical/technotes/statistical_reference_guide.pdf> [retrieved on 2002-08-22]**
• **CHEN Y ET AL: "Ratio-Based Decisions and the Quantitative Analysis of cDNA Microarray Images" JOURNAL OF BIOMEDICAL OPTICS, INTERNATIONAL SOCIETY FOR OPTICAL ENGINEERING (SPIE), US, vol. 2, no. 4, October 1997 (1997-10), pages 364-374, XP002901388 ISSN: 1083-3668**

EP 1 136 933 B1

**Description**

**FIELD OF INVENTION**

This invention is related to bioinformatics and biological data analysis. Specifically, this invention provides methods and systems for the analysis of biological data.

**BACKGROUND OF THE INVENTION**

Many biological functions are carried out by regulating the expression levels of various genes, either through changes in the copy number of the genetic DNA, through changes in levels of transcription (*e.g.* through control of initiation, provision of RNA precursors, RNA processing, *etc.*) of particular genes, or through changes in protein synthesis. For example, control of the cell cycle and cell differentiation, as well as diseases, are characterized by the variations in the transcription levels of a group of genes.

Recently, massive parallel gene expression monitoring methods have been developed to monitor the expression of a large number of genes using nucleic acid array technology which was described in detail in, for example, U. S. Patent Number 5,871,928; de Saizieu, *et al.*, 1998, Bacteria Transcript Imaging by Hybridization of total RNA to Oligonucleotide Arrays, NATURE BIOTECHNOLOGY, 16:45-48; Wodicka *et al.*, 1997, Genome-wide Expression Monitoring in *Saccharomyces cerevisiae*, NATURE BIOTECHNOLOGY 15:1359-1367; Lockhart *et al.*, 1996, Expression Monitoring by Hybridization to High Density Oligonucleotide Arrays. NATURE BIOTECHNOLOGY 14:1675-1680; Lander, 1999, Array of Hope, NATURE-GENETICS, 21(suppl.), at 3. Chen *et al.* (JOURNAL OF BIOMEDICAL OPTICS, International Society for Optical Engineering, Vol. 2, 1997, 364-374) used the Mann-Whitney hypothesis test in a pixel selection method and stated that it would be inappropriate to pool statistics on gene expression differences across the microarray.

Massive parallel gene expression monitoring experiments generate unprecedented amounts of information. For example, a commercially available GeneChip® array set is capable of monitoring the expression levels of approximately 6,500 murine genes and expressed sequence tags (ESTs) (Affymetrix, Inc, Santa Clara, CA, USA). Array sets for approximately 60,000 human genes and EST clusters, 24,000 rat transcripts and EST clusters and arrays for other organisms are also available from Affymetrix. Effective analysis of the large amount of data may lead to the development of new drugs and new diagnostic tools. Therefore, there is a great demand in the art for methods for organizing, accessing and analyzing the vast amount of information collected using massive parallel gene expression monitoring methods.

**SUMMARY OF THE INVENTION**

The current invention provides methods and systems for analyzing data from gene expression monitoring experiments that employ multiple probes against a single target.

In one aspect of the invention, non-parametric statistical analysis is employed to analyze results of multiple probe gene expression experiments with control probes such as mismatch probes. In some embodiments, each target sub-region of a transcript is detected using two probes. One of the probes is a perfect match (PM) probe that is designed to be completely complementary to a reference or target transcript. A mismatch (MM) probe is a probe that is designed to be complementary to a reference sequence except for some mismatches that may significantly affect the hybridization between the probe and its target sequence. In preferred embodiments, MM probes are designed to be complementary to a reference sequence except for a homomeric base mismatch at the central (e.g., 13th in a 25 base probe) position. Mismatch probes are normally used as controls for cross-hybridization. A probe pair is usually composed of a PM and its corresponding MM probe. The difference between PM and MM provides an intensity difference in a probe pair.

In one aspect of the invention, computer implemented methods are used for determining whether a transcript is present in a biological sample. The methods include the steps of providing a plurality of perfect match intensity values ($PM_i$) and mismatch intensity values ($MM_i$) for the transcript, where each of the $PM_i$ is paired with one of the $MM_i$; calculating a *p*-value using one sided Wilcoxon's signed rank test, where the *p*-value is for a null hypothesis that $\theta = a$ threshold value and an alternative hypothesis that said $\theta >$ the threshold value, wherein said $\theta$ is a test statistic for intensity difference between the perfect match intensity values and, mismatch intensity values; and indicating whether the transcript is present based upon the *p*-value.

In some embodiments, the testing statistic is *median($PM_i$-$MM_i$)*. The threshold value may be zero. In some preferred embodiments, the threshold value is calculated using $\tau_1 = c_1 \sqrt{median(PM_i)}$ wherein said $c_1$ is a constant. Alternatively, the threshold value is calculated using: $\tau_1 = c_1 \sqrt{mean(PM_i)}$ wherein $c_1$ is a constant.

The presence, marginal presence or absence (detected, marginally detected or undetected) of a transcript may be called based upon the *p*-value and significance levels. Significance levels, $\alpha_1$ and $\alpha_2$ may be set such that: $0<\alpha_1<\alpha_2<0.5$. Note that for the one-sided test, if the null hypothesis is true, the most likely observed *p*-value is 0.5, which is equivalent to 1 for the two-sided test. Let *p* be the *p*-value of one sided signed rank test. In preferred embodiments, if $p<\alpha_1$, a "detected" call can be made (i.e., the expression of the target gene is detected in the sample). If $\alpha_1 \leq p <\alpha_2$, a marginally detected call may be made. If $p \geq \alpha_2$, an "undetected call" may be made. The proper choice of significance levels and the thresholds can reduce false calls. In some preferred embodiments, $0<\alpha_1<\alpha_2<0.06$. In some particularly preferred embodiments, $\alpha_1$ is around 0.04 and $\alpha_2$ is around 0.06.

In some particularly preferred embodiments, the testing statistic is $median((PM_i-MM_i)/(PM_i+MM_i))$. In these embodiments, the threshold value is a constant. Typically, the threshold value is around 0.001 to 0.05. Most preferably, the threshold value is around 0.015.

In another aspect of the invention, computer implemented methods are provided for analyzing gene expression experiments where a transcript is detected with multiple probes. The method include steps of providing a plurality of perfect match intensity values ($PM_i$) and background intensity values ($B_i$) for the transcript, where each of the $PM_i$ is paired with its corresponding $B_i$; calculating a *p* value using one sided Wilcoxon's signed rank test, wherein the *p* value is for a null hypothesis that $\theta$=a threshold value and an alternative hypothesis that the $\theta>$ the threshold value, where the $\theta$ is a test statistic for intensity difference between the perfect match intensity values and background intensity values; and indicating whether the transcript is present based upon the *p*-value. In preferred embodiments, the testing statistic is $median(PM_i-B_i)$.

The threshold value can be zero. However, in preferred embodiments, the threshold value is calculated using $\tau_3 = c_3\sqrt{median(PM_i)}$ where the $c_3$ is a constant. Alternatively, the threshold value is calculated using: $\tau_3 = c_3\sqrt{mean(PM_i)}$ where the $c_3$ is a constant.

The presence, marginal presence or absence (detected, marginally detected or undetected) of a transcript may be called based upon the *p*-value and significance levels. Significance levels, $\alpha_1$ and $\alpha_2$ may be set such that: $0<\alpha_1<\alpha_2<0.5$. Note that for the one-sided test, if the null hypothesis is true, the most likely observed *p*-value is 0.5, which is equivalent to 1 for the two-sided test. Let *p* be the *p*-value of one-sided signed rank test. In preferred embodiments, if $p<\alpha_1$, a "detected" call can be made (i.e., the expression of the target gene is detected in the sample). If $\alpha_1 \leq p <\alpha_2$, a marginally detected call may be made. If $p \geq \alpha_2$, an "undetected call" may be made. The proper choice of significance levels and the thresholds can reduce false calls. In some preferred embodiments, $0<\alpha_1<\alpha_2<0.06$. In some particularly preferred embodiments, $\alpha_1$ is around 0.04 and $\alpha_2$ is around 0.06.

In addition, systems for determining whether a transcript is present in a biological sample are also provided. The systems include a processor; and a memory being coupled to the processor, the memory storing a plurality of machine instructions that cause the processor to perform a plurality of logical steps when implemented by the processor; the logical steps including the method steps of the invention.

## BRIEF DESCRIPTION OF THE DRAWINGS

The accompanying drawings, which are incorporated in and form a part of this specification, illustrate embodiments of the invention and, together with the description, serve to explain the principles of the invention:

Figure 1 illustrates an example of a computer system that may be utilized to execute the software of an embodiment of the invention.

Figure 2 illustrates a system block diagram of the computer system of Fig. 1.

Figure 3 is a schematic showing a set of probes with 20 probe pairs.

Figure 4 is a schematic showing a computerized method for detecting transcript.

Figure 5 is a schematic showing a process for detecting a transcript using a statistic *median* ($PM_i-MM_i$).

Figure 6 is a schematic showing a process for detecting a transcript using a statistic $median((PM_i-MM_i)/(PM_1+MM_i))$.

Figure 7 is a schematic showing a process for detecting a transcript without using mismatch intensity values,

Figure 8 is a schematic showing a process for detecting a large number of transcripts.

## DETAILED DESCRIPTION OF THE PREFERRED EMBODIMENTS

Reference will now be made in detail to the preferred embodiments of the invention.

## I. Gene Expression Monitoring With High Density Oligonucleotide Probe Arrays

High density nucleic acid probe arrays, also referred to as "DNA Microarrays," have become a method of choice for monitoring the expression of a large number of genes. As used herein, "Nucleic acids" may include any polymer or oligomer of nucleosides or nucleotides (polynucleotides or oligonucleotidies), which include pyrimidine and purine bases, preferably cytosine, thymine, and uracil, and adenine and guanine, respectively. See Albert L Lehninger, PRINCIPLES OF BIOCHEMISTRY, at 793-800 (Worth Pub. 1982) and L. Stryer BIOCHEMISTRY, 4th Ed., (March 1995). "Nucleic acids" may include any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

"A target molecule" refers to a biological molecule of interest. The biological molecule of interest can be a ligand, receptor, peptide, nucleic acid (oligonucleotide or polynucleotide of RNA or DNA), or any other of the biological molecules listed in U.S. Patent No. 5,445,934 at col. 5, line 66 to col. 7, line 51. For example, if transcripts of genes are the interest of an experiment, the target molecules would be the transcripts. Other examples include protein fragments, small molecules, etc. "Target nucleic acid" refers to a nucleic acid (often derived from a biological sample) of interest. Frequently, a target molecule is detected using one or more probes. As used herein, a "probe" is a molecule for detecting a target molecule. It can be any of the molecules in the same classes as the target referred to above. A probe may refer to a nucleic acid, such as an oligonucleotide, capable of binding to a target nucleic acid of complementary sequence through one or more types of chemical bonds, usually through complementary base pairing, usually through hydrogen bond formation. As used herein, a probe may include natural (i.e. A, G, U, C, or T) or modified bases (7-deazaguanosine, inosine, etc.). In addition, the bases in probes may be joined by a linkage other than a phosphodiester bond, so long as the bond does not interfere with hybridization. Thus, probes may be peptide nucleic acids in which the constituent bases are joined by peptide bonds rather than phosphodiester linkages. Other examples of probes include antibodies used to detect peptides or other molecules, any ligands for detecting its binding partners. When referring to targets or probes as nucleic acids, it should be understood that there are illustrative embodiments that are not to limit the invention in any way.

In preferred embodiments, probes may be immobilized on substrates to create an array. An "array" may comprise a solid support with peptide or nucleic acid or other molecular probes attached to the support. Arrays typically comprise a plurality of different nucleic acids or peptide probes that are coupled to a surface of a substrate in different, known locations. These arrays, also described as "microarrays" or colloquially "chips" have been generally described in the art, for example, in Fodor et al., Science, 251:767-777 (1991). Methods of forming high density arrays of oligonucleotides, peptides and other polymer sequences with a minimal number of synthetic steps are disclosed in, for example, 5,143,854, 5,252,743, 5,384,261, 5,405,783, 5,424,186, 5,429,807, 5,445,943, 5,510,270, 5,677,195, 5,571,539, 6,044,138. The oligonucleotide analogue array can be synthesized on a solid substrate by a variety of methods, including, but not limited to, light-directed chemical coupling, and mechanically directed coupling. See Pirrung et al., U.S. Patent No. 5,143,854 (see also PCT Application No. WO 90/15070) and Fodor et al., PCT Publication Nos. WO 92/10092 and WO 93/09668, U.S. Pat. Nos. 5,677,195, 5,800,992 and 6,156,501 which disclose methods of forming vast arrays of peptides, oligonucleotides and other molecules using, for example, light-directed synthesis techniques. See also, Fodor et al., Science, 251, 767-77 (1991). These procedures for synthesis of polymer arrays are now referred to as VLSIPS™ procedures. Using the VLSIPS™ approach, one heterogeneous array of polymers is converted, through simultaneous coupling at a number of reaction sites, into a different heterogeneous array. See, U. S. Patent Nos. 5,384,261 and 5,677,195.

Methods for making and using molecular probe arrays, particularly nucleic acid probe arrays are also disclosed in, for example, U.S. Patent Numbers 5,143,854, 5,242,974, 5,252,743, 5,324,633, 5,384,261, 5,405,783, 5,409,810, 5,412,087, 5,424,186, 5,429,807, 5,445,934, 5,451,683, 5,482,867, 5,489,678, 5,491,074, 5,510,270, 5,527,681, 5,527,681, 5,541,061, 5,550,215, 5,554,501, 5,556,752, 5,556,961, 5,571,639, 5,583,211, 5,593,839, 5,599,695, 5,607,832, 5,624,711, 5,677,195, 5,744,101, 5,744,305, 5,753,788, 5,770,456, 5,770,722, 5,831,070, 5,856,101, 5,885,837, 5,889,165, 5,919,523, 5,922,591, 5,925,517, 5,658,734, 6,022,963, 6,150,147, 6,147,205, 6,153,743, 6,140,044 and D430024. Typically, a nucleic acid sample is labeled with a signal moiety, such as a fluorescent label. The sample is hybridized with the array under appropriate conditions. The arrays are washed or otherwise processed to remove non-hybridized sample nucleic acids. The hybridization is then evaluated by detecting the distribution of the label on the chip. The distribution of label may be detected by scanning the arrays to determine florescence intensities distribution. Typically, the hybridization of each probe is reflected by several pixel intensities. The raw intensity data may be stored in a gray scale pixel intensity file. The GATC™ Consortium has specified several file formats for storing array intensity data. The final software specification is available at www.gatcconsortium.org The pixel intensity files are usually large. For example, a GATC™ compatible image file may be approximately 50 Mb if there are about 5000 pixels on each of the horizontal and vertical axes and if a two byte integer is used for every pixel intensity. The pixels may be grouped into cells (see, GATC™ software specification). The probes in a cell are designed to have the same sequence (i.e., each cell is a probe area). A CEL file contains the statistics of a cell, e.g., the 75 percentile and standard deviation of intensities of pixels in a cell. The 75 percentile of pixel intensity of a cell is often used as the intensity of the cell. Methods for signal detection and processing of intensity data are additionally disclosed in, for example, U.S. Patents Numbers 5,547,839, 5,578,832, 5,631,734, 5,800,992, 5,856,092, 5,936,324, 5,981,956, 6,025,601, 6,090,555, 6,141,096, 6,141,096, and 5,902,723. Methods for array based assays, computer software for data analysis and applications are additionally disclosed in, e.g., U.S. Patent Numbers 5,527,670, 5,527,676, 5,545,531, 5,622,829, 5,631,128, 5,639,423, 5,646,039, 5,650,268, 5,654,155, 5,674,742, 5,710,000, 5,733,729, 5,795,716, 5,814,450, 5,821,328, 5,824,477, 5,834,252, 5,834,758, 5,837,832, 5,843,655, 5,856,086, 5,856,104, 5,856,174, 5,858,659, 5,861,242, 5,859,244, 5,871,928, 5,874,219, 5,902,723, 5,925,525, 5,928,905, 5,935,793, 5,945,334, 5,959,098, 5,968,730, 5,968,740, 5,974,164, 5,981,174, 5,981,185, 5,985,651, 6,013,440, 6,013,449, 6,020,135, 6,027,880, 6,027,894, 6,033,850, 6,033,860, 6,037,124, 6,040,138, 6,040,193, 6,043,080, 6,045,996, 6,050,719, 6,066,454, 6,083,697, 6,114,116, 6,114,122, 6,121,048, 6,124,102, 6,130,046, 6,132,580, 6,132,996 and 6,136,269.

Nucleic acid probe array technology, use of such arrays, analysis array based experiments, associated computer software, composition for making the array and practical applications of the nucleic acid arrays are also disclosed, for example, in the following U.S. Patent Applications: 07/838,607, 07/883,327, 07/978,940, 08/030,138, 09/418,946, 09/420,805, 09/428,350, 09/431,964, 09/445,734, 09/464,350, 09/475,209, 09/502,048, 09/510,643, 09/513,300, 09/516,388, 09/528,414, 09/535,142, 09/544,627, 09/620,780, 09/640,962, 09/641,081, 09/670,510, 09/685,011, and 09/693,204 and in the following Patent Cooperative Treaty (PCT) applications/publications: PCT/NL90/00081, PCT/ GB91/00066, PCT/US91/08693, PCT/US91/09226, PCT/US91/09217, WO/93/10161, PCT/US92/10183, PCT/ GB93/00147, PCT/US93/01152, WO/93/22680, PCT/US93/04145, PCT/US93/08015, PCT/US94/07106, PCT/ US94/12305, PCT/GB95/00542, PCT/US95/07377, PCT/US95/02024, PCT/US96/05480, PCT/US96/11147, PCT/ US96/14839, PCT/US96/15606, PCT/US97/01603, PCT/US97/02102, PCT/GB97/005566, PCT/US97/06535, PCT/ GB97/01148, PCT/GB97/01258, PCT/US97/08319, PCT/US97/08446, PCT/US97/10365, PCT/US97/17002, PCT/ US97/16738, PCT/US97/19665, PCT/US97/20313, PCT/US97/21209, PCT/US97/21782, PCT/US97/23360, PCT/ US98/06414, PCT/US98/01206, PCT/GB98/00975, PCT/US98/04280, PCT/US98/04571, PCT/US98/05438, PCT/ US98/05451, PCT/US98/12442, PCT/US98/12779, PCT/US98/12930, PCT/US98/13949, PCT/US98/15151, PCT/ US98/15469, PCT/US98/15458, PCT/US98/15456, PCT/US98/16971, PCT/US98/16686, PCT/US99/19069, PCT/ US98/18873, PCT/US98/18541, PCT/US98/19325, PCT/US98/22966, PCT/US98/26925, PCT/US98/27405 and PCT/ IB99/00048. PCT/US98/05438, PCT/US98/05451, PCT/US98/12442, PCT/US98/12779, PCT/US98/12930, PCT/ US98/13949, PCT/US98/15151, PCT/US98/15469, PCT/US98/15458, PCT/US98/15456, PCT/US98/16971, PCT/ US98/16686, PCT/US99/19069, PCT/US98/18873, PCT/US98/18541, PCT/US98/19325, PCT/US98/22966, PCT/ US98/26925, PCT/US98/27405 and PCT/IB99/00048.

The embodiments of the invention will be described using GeneChip® high density oligonucleotide probe arrays (available from Affymetrix, Inc., Santa Clara, CA, USA) as exemplary embodiments. One of skill the art would appreciate that the embodiments of the invention are not limited to high density oligonucleotide probe arrays. In contrast, the embodiments of the invention are useful for analyzing any parallel large scale biological analysis, such as those using nucleic acid probe array, protein arrays, etc.

Gene expression monitoring using GeneChip® high density oligonucleotide probe arrays are described in, for example, Lockhart et al., 1996, Expression Monitoring By Hybridization to High Density Oligonucleotide Arrays, Nature Biotechnology 14:1675-1680; U.S. Patent Nos. 6,040,138 and 5,800,992.

In the preferred embodiment, oligonucleotide probes are synthesized directly on the surface of the array using photolithography and combinatorial chemistry as disclosed

in several patents. In such embodiments, a single square-shaped feature on an array contains one type of probe. Probes are selected to be specific against desired target. Methods for selecting probe sequences are disclosed in, for example, U.S. Patent Application Nos. 09/718,295, 09/721,042, and 60/252,617.

In a preferred embodiment, oligonucleotide probes in the high density array are selected to bind specifically to the nucleic acid target to which they are directed with minimal non-specific binding or cross-hybridization under the particular hybridization conditions utilized. Because the high density arrays of this invention can contain in excess of 1,000,000 different probes, it is possible to provide every probe of a characteristic length that binds to a particular nucleic acid sequence. Thus, for example, the high density array can contain every possible 20 mer sequence complementary to an IL-2 mRNA. There, however, may exist 20 mer subsequences that are not unique to the IL-2 mRNA. Probes directed to these subsequences are expected to cross hybridize with occurrences of their complementary sequence in other regions of the sample genome. Similarly, other probes simply may not hybridize effectively under the hybridization conditions (e.g., due to secondary structure, or interactions with the substrate or other probes). Thus, in a preferred embodiment, the probes that show such poor specificity or hybridization efficiency are identified and may not be included either in the high density array itself (e.g., during fabrication of the array) or in the post-hybridization data analysis.

Probes as short as 15, 20, 25 or 30 nucleotides are sufficient to hybridize to a subsequence of a gene and that, for most genes, there is a set of probes that performs well across a wide range of target nucleic acid concentrations.

In a preferred embodiment, it is desirable to choose a preferred or "optimum" subset of probes for each gene before synthesizing the high density array.

In some preferred embodiments, the expression of a particular transcript may be detected by a plurality of probes, typically, up to 5, 10, 15, 20, 30 or 40 probes. Each of the probes may target different sub-regions of the transcript. However, probes may overlap over targeted regions.

In some preferred embodiments, each target sub-region is detected using two probes: a perfect match (PM) probe that is designed to be completely complementary to a reference or target sequence. In some other embodiments, a PM probe may be substantially complementary to the reference sequence: A mismatch (MM) probe is a probe that is designed to be complementary to a reference sequence except for some mismatches that may significantly affect the hybridization between the probe and its target sequence. In preferred embodiments, MM probes are designed to be complementary to a reference sequence except for a homomeric base mismatch at the central (e.g., 13$^{th}$ in a 25 base probe) position. Mismatch probes are normally used as controls for cross-hybridization. A probe pair is usually composed of a PM and its corresponding MM probe. The difference between PM and MM provides an intensity difference in a probe pair.

## II. Data Analysis Systems

In one aspect of the invention, methods and systems are provided for computational analysis of microarray intensity data for determining the presence or absence of genes in a given biological sample. Software written for the present invention is to be stored in some form of computer readable medium, such as memory, or CD-ROM, or transmitted over a network, and executed by a processor. For a description of basic computer systems and computer networks, see, e.g., Introduction to Computing Systems: From Bits and Gates to C and Beyond by Yale N. Patt, Sanjay J. Patel, 1st edition (January 15, 2000) McGraw Hill Text; ISBN: 0072376902; and Introduction to Client/Server Systems: A Practical Guide for Systems Professionals by Paul E. Renaud, 2nd edition (June 1996), John Wiley & Sons; ISBN: 0471133337.

Computer software products may be written in any of various suitable programming languages, such as C, C++, C# (Microsoft®), Fortran, Perl, MatLab (MathWorks, www.mathworks.com), SAS, SPSS and Java. The computer software product may be an independent application with data input and data display modules. Alternatively, the computer software products may be classes that may be instantiated as distributed objects. The computer software products may also be component software such as Java Beans (Sun Microsystem), Enterprise Java Beans (EJB, Sun Microsystems), Microsoft® COM/DCOM (Microsoft®), etc.

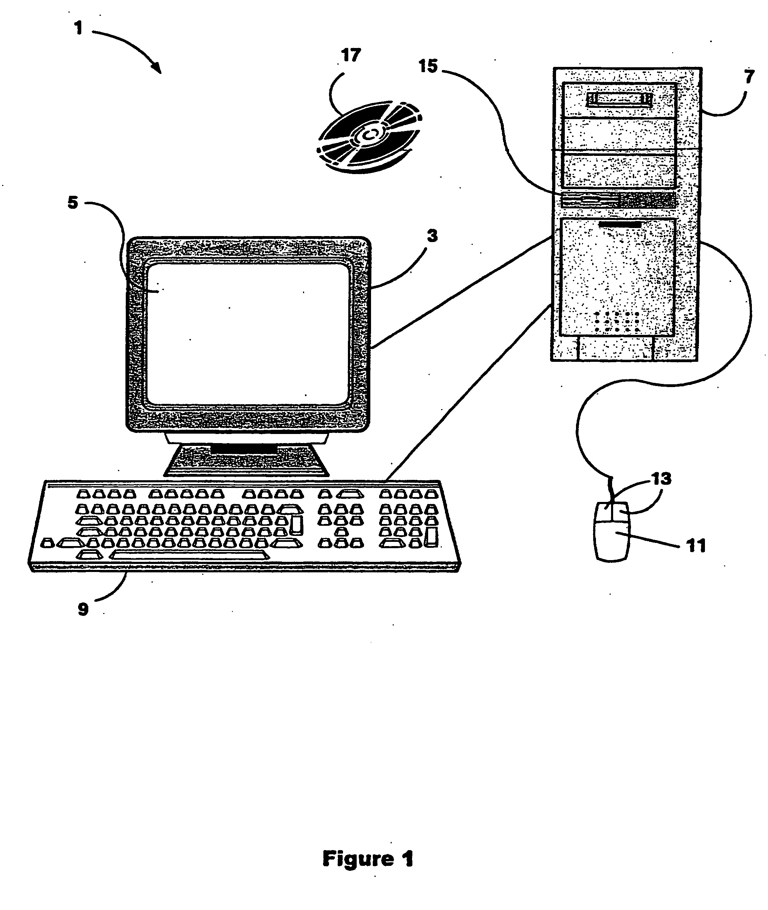

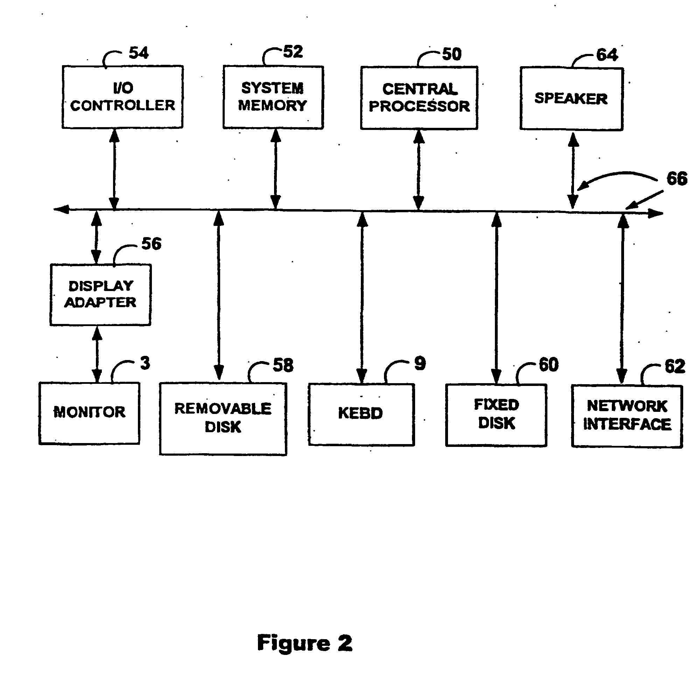

Figure 1 illustrates an example of a computer system that may be used to execute the software. Figure 1 shows a computer system 1 that includes a display 3, screen 5, cabinet 7, keyboard 9, and mouse 11. Mouse 11 may have one or more buttons for interacting with a graphic user interface. Cabinet 7 houses a CD-ROM or DVD-ROM drive 13, system memory and a hard drive (*see* Figure 2) which may be utilized to store and retrieve software programs incorporating computer code that implements the invention, data for use with the invention and the like. Although a CD 15 is shown as an exemplary computer readable medium, other computer readable storage media including floppy disk, tape, flash memory, system memory, and hard drive may be utilized. Additionally, a data signal embodied in a carrier wave (*e.g.*, in a network including the Internet) may be the computer readable storage medium.

Figure 2 shows a system block diagram of computer system 1 used to execute the software of an embodiment of the invention. As in Figure 1, computer system 1 includes monitor 3, keyboard 9, and mouse 11. Computer system 1 further includes subsystems such as a central processor 51, system memory 53, fixed storage 55 (*e.g.*, hard drive), removable storage 57 (*e.g.*, CD-ROM), display adapter 59, sound card 61, speakers 63, and network interface 65. Other computer systems suitable for use with the invention may include additional or fewer subsystems. For example, another computer system may include more than one processor 51 or a cache memory: Computer systems suitable for use with the invention may also be embedded in a measurement instrument.

## III. Gene Expression Calls Using Non-Parametric Statistics

Computational analysis of probe array intensity data for determining the presence or absence of expression of genes in a given biological sample is a crucial step in extraction of useful information from experimental data. There may be a large uncertainty associated with these calls (i.e., determination of the presence or absence of the expression of genes) because of many random effects such as inevitable variations in manufacturing and experimental conditions, and the complexity of cross hybridization.

It is therefore desirable for every call to provide a p-value, the probability value for a testing statistic be equal or further extreme to its observed value under the null hypothesis.

In one aspect of the invention, methods and systems are provided to determine (or call) the presence or absence of the expression of target genes using data from gene expression experiments that employ multiple probes against a single target. The methods include steps for computing *p*-values of such calls using non-parametric statistics.

Nonparametric statistical methods are powerful tools for computing exact *p*-values when the distribution of original data is unknown (e.g., Wilcoxon, F. *Individual Comparisons. by Ranking Methods*, Biometrics, 1:80-83 (1945), Hogg RV, Tanis EA (1997) *Probability and Statistical Inference* (fifth edition), Upper Saddle River, NJ:Prentice-Hall, Inc.; Hollander M, Wolfe DA (1999). *Nonparametric Statistical Methods* (second edition), New York: John Wiley & Sons, Inc.

Many nonparametric methods use ranks or signs of data, and hence are insensitive to outliers. Their assumptions about the distributions of the original data are much weaker than those of parametric methods. Therefore, they can be applied to more general situations.

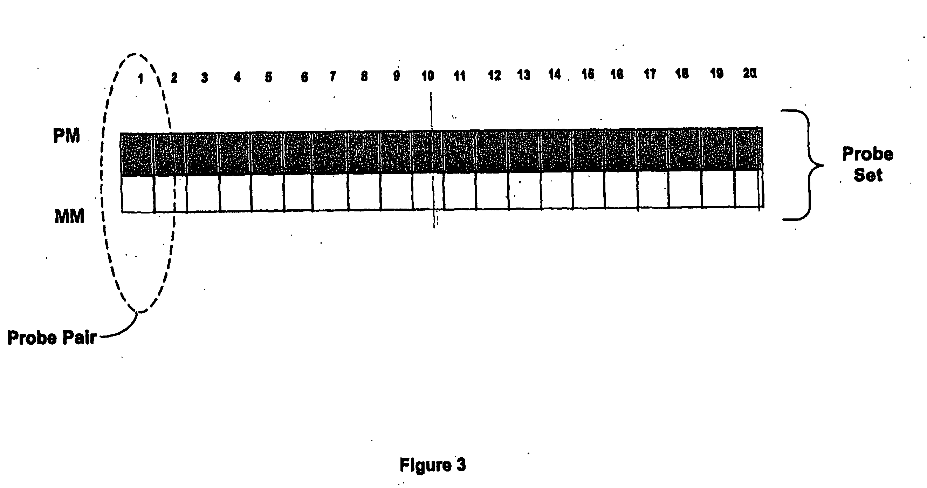

In some embodiments, Wilcoxon's signed rank test is used to analyze paired PM and MM probes. In a block of *n* probe pairs (also known as atoms, Figure 3) for detecting a gene (typically 10,15, or 20 probe pairs). Each probe pair typically consists of two cells, one has the sequence designed to be perfectly matching the target sequence and the other has the sequence designed to be mismatching the target sequence, preferrably at only a single nucleotide location (usually at the center of the sequence segment).

Let the *i*-th perfectly matching cell intensity be $PM_i$ and the *i*-th mismatching cell intensity be $MM_i$ ($i=1,....,n$). All these data are positive numbers. As described above, in some embodiments, the hybridization of each probe may be reflected by several pixel intensities. In such embodiments, the cell intensity is derived from the pixel intensities. In preferred embodiments, around 60, 70, 75, 80, 85, or 90 percentile of of intensities of inner pixels in a cell is used to represent the cell intensity. In a particularly preferred embodiment, the 75 percentile of intensities of inner pixels in a cell is used to represent the cell intensity and is saved in a CEL file together with the number of pixels and the standard deviation of intensities at these pixels.

The traditional parametric approach to compare $PM_i$ and $MM_i$ is to test whether the mean of $PM_i$ is equal to the mean of $MM_i$, or equivalently, whether the mean of differences

$$D_i = PM_i - MM_i \tag{1}$$

is zero. To calculate the *p*-value, normal distribution is assumed.

The nonparametric approach, by contrast, is to test whether the median of $PM_i$ is the same as the median of $MM_i$, or equivalently, whether the median of differences $D_i = PM_i - MM_i$ is zero. In practice, it is better to include a positive threshold $\tau$, and to test whether the median of $D_i$ is larger than $\tau$.

In practice; when using differences $D_i$, we require $D_i$ be larger than a certain threshold $\tau$ to make a detected call. Using a threshold $\tau$ can avoid a detected call if most $PM_i's$ are only slightly larger than $MM_i's$, which is possible sometimes when the gene is really absent. In one embodiment, $\tau$ may be calculated as follows:

$$\tau = \frac{c}{2n} \sum_{j=1}^{2n} \frac{s_j}{\sqrt{n_j}}, \tag{2}$$

Other statistics such as the discrimination score (also referred to as Ryder's discrimination score):

$$R_i = \frac{PM_i - MM_i}{PM_i + MM_i} \tag{3}$$

can also be used. In spite of these different flavors of implementation, the principles of computing *p*-values are the same. The methods of the invention involve the testing the null hypothesis

$$H_o: \text{median}(D_i) = 0 \text{ (the gene is not expressed)}$$

against the one-sided upper-tail alternative hypothesis

$$H_1: \text{median}(D_i) > 0. \text{ (the gene is expressed)}$$

Or more rigidly, assume $D_i$'s ($i = 1, ..., n$) are from continuous populations, and are mutually independent. Moreover, the distribution functions $F_i$'s of $D_i$'s are symmetric about a common median $\theta$, i.e.,

$$F_i(\theta + \chi) + F_i(\theta - \chi) = 1, \tag{4}$$

for all possible values of $\chi$ and $i$. The Wilcoxon's signed rank test can be used to test the null hypothesis

$$H_o: \theta = 0$$

against the one-sided upper-tail alternative hypothesis

$$H_1: \theta > 0.$$

If all values if $D_i$'s are zeros, then we accept the null hypothesis and let the $p$-value be 0.5 for the one-sided test. If there are some but not all values of $D_i$'s are zeros, then we remove these zero values and adjust the block size $n$ to be the number of nonzero $D_i$'s that are used for computing $p$-values.

Where there are no zero values of $D_i$'s, all $|D_i|$'s are sorted in the ascending order. If there are no ties among $|D_i|$'s, they can be ranked and assigned positive integers $1,2,...,n$. Then, the signs of the original data are given to the ranks.

If there are ties of $|D_i|$'s the average of the consecutive integer ranks are assigned to entries in a tied group. For example, consider six probe pairs with $D_1 = 150.2$, $D_2 = -300.1$, $D_3 = 1700.1$, $D_4 = -150.2$, $D_5 = -50.3$, $D_6 = -50.3$. There are two tied groups: $|D_1| = |D_4|$ and $|D_5| = |D_6|$. The signed ranks are $r_1 = 3.5$, $r_2 = -5$, $r_3 = 6$, $r_4 = 3.5$, $r_5 = -1.5$, and $r_6 = -1.5$.

Let signed rank of $D_i$ be $r_i$. The sum of all positive signed ranks may be calculated as follows:

$$W_1{}^+ = \sum_{i=1}^{n} c_i r_i, \tag{5}$$

where the characteristic coefficient

$$c_i = \begin{cases} 1, & \text{if } r_i > 0, \\ 0, & \text{if } r_i < 0. \end{cases} \tag{6}$$

Under the null hypothesis, all $2^n$ possible patterns of signed ranks are uniformly distributed. Using this fact, the exact $p$-value for an observed $W_1{}^+$ statistic can be calculated.

The computation of exact $p$-value becomes expensive when $n$ is large. For large samples, under the null hypothesis, normal approximation for $W_1{}^+$ may be used, whose mean and variance are respectively

$$\mu_{W_1}{}^+ = \frac{n(n + 1)}{4}, \tag{7}$$

$$V_{W_1}{}^+ = \frac{1}{24}\left[ n(n+1)(2n+1) - \frac{1}{2}\sum_{k=1}^{g} t_k(t^2{}_k - 1) \right], \tag{8}$$

where $g$ is the number of tied groups, and $t_k$ is the number of tied entries in tied group $k$. Then the statistic

$$W_1^* = \frac{W_1^+ - \mu_{w_1^+}}{\sqrt{V_{w_1^+}}} \qquad (9)$$

should approximately have the standard normal distribution $N(0,1)$.

Statistics other than $D_i = PM_i - MM_i$ can also be used for nonparametric methods. For example, Ryder's discrimination score

$$R_i = \frac{PM_i - MM_i}{PM_i + MM_i} = 1 - \frac{2}{\frac{PM_i}{MM_i} + 1}. \qquad (10)$$

is a monotonically increasing function of the ratio $PM_i/MM_i$. It changes the range $(0,\infty)$ of $PM_i/MM_i$ to the range $(-1,1)$ of $R_i$. In another embodiment, the following discrimination score may be used:

$$L_i = \frac{PM_i - MM_i}{\sqrt{PM_i + MM_i}} \qquad (11)$$

To compare the results of two experiments on the same chip, it is important to determine a normalization factor. Let $PM_i^{(b)}$ and $MM_i^{(b)}$ be the intensity data of a baseline experiment, and $PM_i^{(e)}$ and $MM_i^{(e)}$ 'be the intensity data of the other experiment.

Wilcoxon's signed rank test can be applied to compare $PM_i^{(b)}$ with $N \cdot PM_i^{(e)}$. $R_i^{(b)}$ may also be directly compared with $R_i^{(e)}$, Ryder's discrimination score of the baseline experiment with that of the other experiment.

Other nonparametric methods can also be used to analyze microarray intensity data. The CEL files give cell intensity which is the 75 percentile of pixel itensities in a cell. When we want to reduce the number of probe sets in a block, using the DAT file to take all pixel intensities into consideration may give more dependable analysis and more reasonable $p$-values.

In some particularly preferred embodiments, the following three statistics of cell intensities can be used to make calls based on one sided Wilcoxon's signed rank test.

The null hypothesis is denoted $H_0$ and alternative hypothesis $H_1$.

(1) $H_0$: median $(PM_i - MM_i) = \tau_1$;

$H_1$: median $(PM_i - MM_i) > \tau_1$

(2) $H_0$: median $(PM_i - MM_i)/(PM_i + MM_i) = \tau_2$;

$H_1$: median $(PM_i - MM_i)/(PM_i + MM_i) > \tau_2$;

(3) $H_0$: median $(PM_i - B_i) = \tau_3$;

$H_1$: median $(PM_i - B_i) > \tau_3$;

Here, the threshold $\tau_1$, $\tau_2$, and $\tau_3$ are non-negative, and $B_i$ in the third method is a space dependent background. Significance levels, $\alpha_1$ and $\alpha_2$ may be set such that: $0 < \alpha_1 < \alpha_2 < 0.5$. Note that for the one-sided test, if null hypothesis is true, the most likely observed $p$ value is 0.5, which is equivalent to 1 for the two-sided test. Let $p$ be the $p$-value of one sided signed rank test. In preferred embodiments, if $p < \alpha_1$, a "detected" call can be made (i.e., the expression of the target gene is detected in the sample). If $\alpha_1 \le p < \alpha_2$, a marginally detected call may be made. If $p \ge \alpha_2$, "undetected call" may be made. The proper choice of significance levels and the thresholds can reduce false calls.

The threshold $\tau_1$ may be set to be proportional to the square root of the sample median or mean of perfect match intensity, i.e.,

$$\tau_1 = c_1\sqrt{median(PM_i)} \tag{13}$$

The Ryder's discrimination score in the second test is a relative measure of the difference between $PM_i$ and $MM_i$. As discussed above, it is a monotonic function of ratio:

$$r_i = {PM_i} \big/ {MM_i},$$

of perfect match and mismatch intensities:

$$R_i = (r_i - 1)/(r_i + 1) \tag{14}$$

Using the Ryder statistic is particularly preferred if both perfect match and mismatch intensities are available. One benefit is that a constant $\tau_2$ may be used to get good results. Another justification of using a positive $\tau_2$ is that the median of $R_i$ is a small positive number in several sets of Latin square experiments (Example 1, *infra*) with over 100 transcripts in each experiment with concentrations 0, 0.25, 0.5, 1, 2, 4, 8, 16, 32, 64, 128, 256, 512, 1024 pM. Table 1 lists the median, 25 percentile, 75 percentile, minimum and maximum of $R_i$ in every concentration group. Table 2 is similar to Table 1, but with human genome complex background. Both data are on chips with lot number 9912072.

When median ($R_i$) is below 0.7, corresponding approximately to the concentration range below 30-60 pM or so, $R_i$ increases when the concentration of the transcript increases. Beyond this range, $R_i$ may decrease as transcript concentration increases, but remains higher than 0.7. Therefore, $R_i$ is not always related to transcript concentration, especially when median ($R_i$) is above 0.7, but for the purpose of absolute calls, *i.e.*, to detect the existence of a gene above a small concentration, *e.g.*, 1 or 2 pM, $R_i$ is a very good statistic to use.

$\tau_2$ may be set according user preference. In preferred embodiment, $\tau_2$ may be set at 0.005-0.05, preferably around 0.015, more preferably at 0.015. The third test, $H_0$: median ($PM_i - B_i$) = $\tau_3$; $H_1$:median ($PM_i - B_i$) > $\tau_3$;

If mismatch intensities are not available, $B_i$ can be considered as a background, e.g., the average of the lowest 2 or the result of smoothing these averages. $B_i$ so calculated is usually lower than the mismatch intensities, but this can be made up by adjusting $\tau_3$. In some cases, $\tau_3$ may be set to be proportional to the square root of the sample median or mean of perfect match intensity, i.e.,

$$\tau_3 = c_3\sqrt{median(PM_i)} \tag{15}$$

When a gene has splicing variants, a subset of atoms in a block can show significant positive differences $D_i$, but within the whole block the differences may not be so significantly different from 0. Nonparametric algorithms can detect this situation by calculating the relevant statistics for subsets of a block of atoms.

The nonparametric algorithms suggested here need sorting. The results of sorting subsets can be used for sorting the whole set with the merge sort algorithm. It can save the total computing time for the detection of splicing variants with nonparametric algorithms.

### IV. Methods and Systems for Making Gene Expression Calls

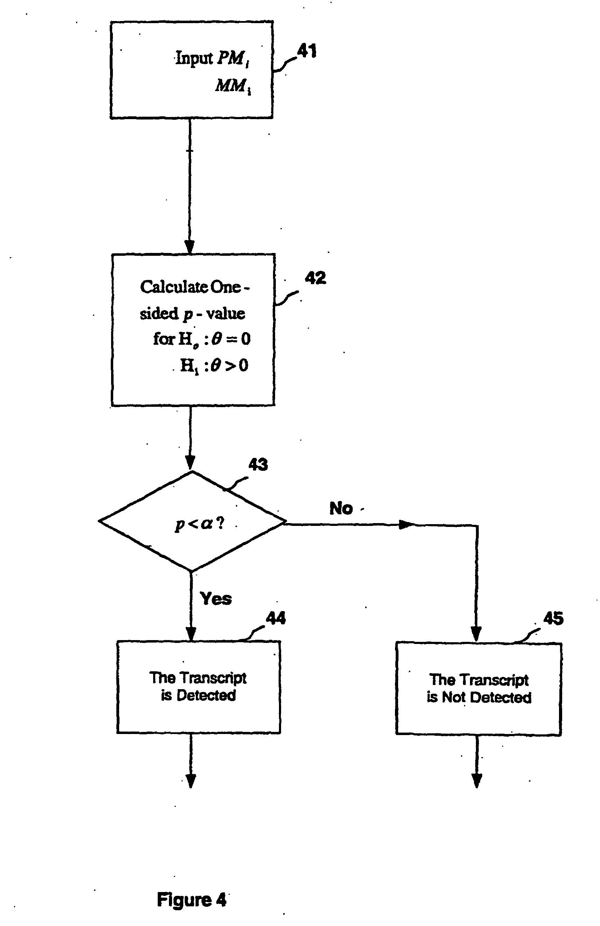

In one aspect of the invention, computer implemented methods are provided for analyzing gene expression experiments. Figure 4 shows an exemplary process of the methods of the invention. Intensity data (such as perfect match intensities ($PM_i$) and mismatch intensities ($MM_i$) from the CEL file) are obtained (41) from data file (such as the CEL file), directly from a measurement instrument (such as a scanner) or other data source. The intensity data are processed to calculate a p-value for the following hypotheses:$H_0$: $\theta = \tau$; $H_1$: $\theta > \tau$, where $\theta$ is a test statistic for intensity difference between the perfect match intensity values and the mismatch intensity values. The *p*-value is calculated using one-sided Wilcoxon's signed rank test.

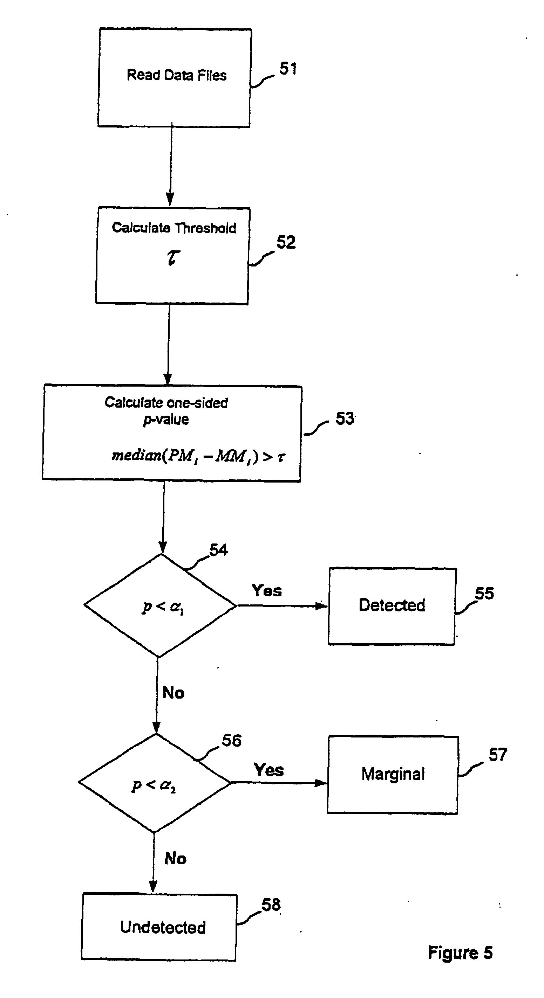

In some embodiments (Figure 5), the testing statistic is *median(PM_i-MM_i)* (53). The threshold value may be zero. In some preferred embodiments, the threshold value is calculated (52) using: $\tau_1 = c_1\sqrt{median(PM_i)}$ wherein said

$c_1$ is a constant. Alternatively, the threshold value is calculated using: $\tau_1 = c_1\sqrt{mean(PM_i)}$ wherein $c_1$ is a constant.

The presence, marginal presence or absence (detected, marginally detected or undetected) of a transcript may be called based upon the *p*-value and significance levels (54-58). Significance levels, $\alpha_1$ and $\alpha_2$ may be set such that: $0<\alpha_1<\alpha_2<0.5$. Note that for the one-sided test, if the null hypothesis is true, the most likely observed *p*-value is 0.5, which is equivalent to 1 for the two-sided test. Let *p* be the *p*-value of one sided signed rank test. In preferred embodiments, if $p<\alpha_1$, a "detected" call can be made (i.e., the ex-pression of the target gene is detected in the sample). If $\alpha_1 \leq p < \alpha_2$, a marginally detected call may be made. If $p \geq \alpha_2$, an "undetected call" may be made. The proper choice of significance levels and the thresholds can reduce false calls. In some preferred embodiments, $0<\alpha_1<\alpha_2<0.06$. In some particularly preferred embodiments, $\alpha_1$ is around 0.04 and $\alpha_2$ is around 0.06.

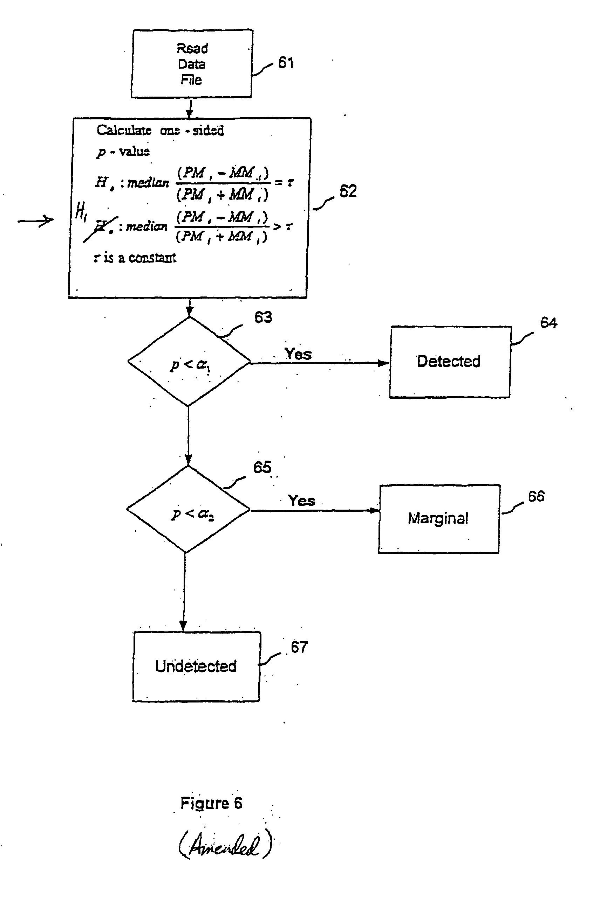

In some particularly preferred embodiments (Figure 6), the testing statistic is $median((PM_i\text{-}MM_i)/(PM_i+MM_i))$. In these embodiments, the threshold value is a constant. Typically, the threshold value is around 0.001 to 0.05. Most preferably, the threshold value is around 0.015.

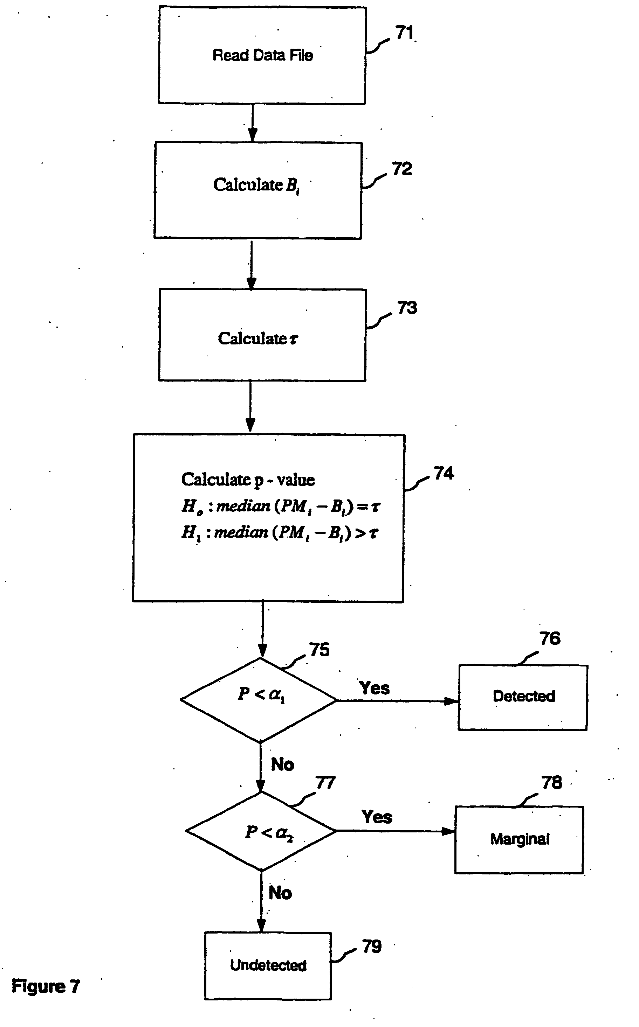

In another aspect of the invention, computer implemented methods are provided for analyzing gene expression experiments where a transcript is detected with multiple probes (Figure 7). The method include steps of providing a plurality of perfect match intensity values ($PM_i$) and background intensity values ($B_i$) for the transcript (71), where each of the $PM_i$ is paired with its corresponding $B_i$; calculating a *p* value using one sided Wilcoxon's signed rank test, wherein the *p* value is for a null hypothesis that $\theta=$a threshold value and an alternative hypothesis that the $\theta>$ the threshold value, where the $\theta$ is a test statistic for intensity difference between the perfect match intensity values and background intensity values; and indicating whether the transcript is present based upon the *p* value. In preferred embodiments, the testing statistic is $median(PM_i\text{-}B_i)$ (74).

The threshold value can be zero. However, in preferred embodiments, the threshold value is calculated using: $\tau_1 = c_1\sqrt{median(PM_i)}$ where the $c_1$ is a constant. Alternatively, the threshold value is calculated using: $\tau_3 = c_3\sqrt{mean(PM_i)}$ where the $c_3$ is a constant.

The presence, marginal presence or absence (detected, marginally detected or undetected) of a transcript may be called based upon the *p* -value and significance levels. Significance levels, $\alpha_1$ and $\alpha_2$ may be set such that: $0<\alpha_1<\alpha_2<0.5$. Note that for the one-sided test, if the null hypothesis is true, the most likely observed *p* value is 0.5, which is equivalent to 1 for the two-sided test. Let *p* be the *p*-value of one sided signed rank test. In preferred embodiments, if $p<\alpha_1$, a "detected" call can be made (i.e., the expression of the target gene is detected in the sample). If $\alpha_1 \leq p < \alpha_2$, a marginally detected call may be made. If $p \geq \alpha_2$, an "undetected call" may be made. The proper choice of significance levels and the thresholds can reduce false calls. In some preferred embodiments, $0<\alpha_1<\alpha_2<0.06$. In some particularly preferred embodiments, $\alpha_1$ is around 0.04 and $\alpha_2$ is around 0.06.

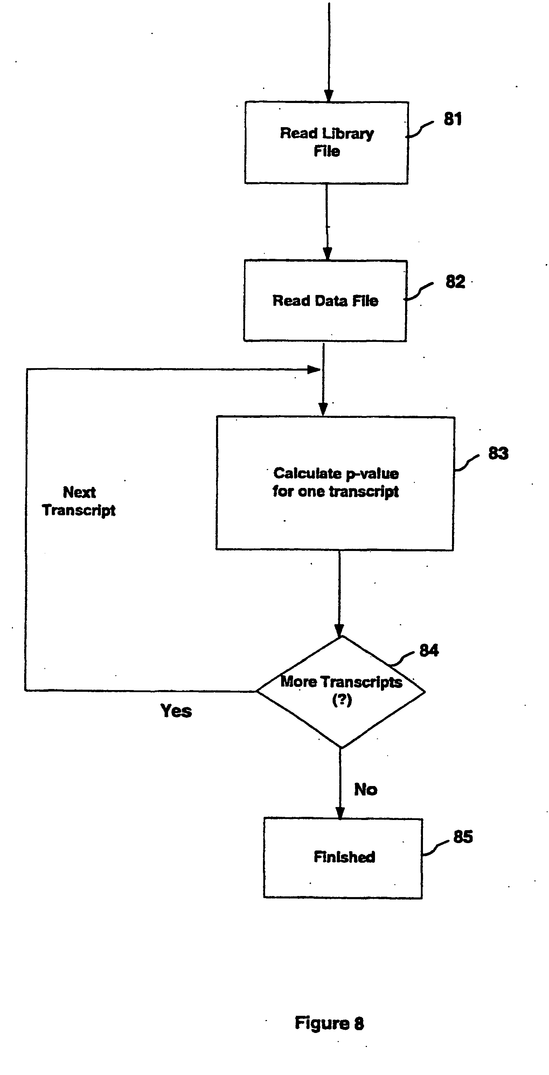

The methods of the invention are particularly suitable for analyzing a large number of transcripts, preferably more than 50, 100, 500, 750, 1000, 2000, 3000, 5000, 10,000 or more. Figure 8 shows a process for detecting the presence of a large number of transcripts. In this embodiment, a library file which can be used to identify the relationship between probe sets and transcripts is read (81). Each of the probe sets may be targeting one transcript. An intensity data file contains intensity for a large number of probe sets (82), such as the CEL file, is also read. The *p*-value for each transcript is calculated (83, 84). The p-value of each transcript is used to detect the presence or absence of the transcript.

In addition, systems for determining whether a transcript is present in a biological sample are also provided. The systems include a processor; and a memory being coupled to the processor, the memory storing a plurality of machine instructions that cause the processor to perform a plurality of logical steps when implemented by the processor; the logical steps including the method steps of the invention.

### V. Examples.

*Example 1.* Table 1 and 2 list Ryder discrimination score in several sets of Latin square experiments with over 100 genes in each experiment with concentrations 0, 0.25, 0.5, 1, 2, 4, 8, 16, 32, 64, 128, 256, 512, 1024 pM.

Table 1 .

| Statisticsof $R_i$ by Concentration in pMol For a Yeast Latin Square Data Set 9912072 | | | |
|---|---|---|---|
| Concentration | Median | Interquartile range | Minimum-maximum range |
| 0 | 0.0029 | [-0.0088, 0.0156] | [-0.0528, 0.3229] |
| 0.25 | 0.0994 | [0.0610, 0.1460] | [0.0195, 0.5016] |

Table 1 .  (continued)

| Statisticsof $R_i$ by Concentration in pMol For a Yeast Latin Square Data Set 9912072 | | | |
|---|---|---|---|
| Concentration | Median | Interquartile range | Minimum-maximum range |
| 0.5 | 0.777 | [0.1247, 0.2486] | [0.0468, 0.6398] |
| 1 | 0.2595 | [0.1789, 0.3474] | [0.0502, 0.6527] |
| 2 | 0.3744 | [0.3035, 0.4577] | [0.0892, 0.7533] |
| 4 | 0.5037 | [0.4125, 0.5717] | [0.396, 0.7086] |
| 8 | 0.6138 | [0.5061, 0.6749] | [0.2608, 0.7672] |
| 16 | 0.6704 | [0.6057, 0.7268] | [0.4 53, 0.8166] |
| 32 | 0.7315 | [0.6604, 0.7739] | [0.5358, 0.8418] |
| 64 | 0.7475 | [0.6717, 0.7952] | [0.4562, 0.8464] |
| 128 | 0.7459 | [0.6792, 0.8075] | [0.5772, 0.8611] |
| 256 | 0.7630 | [0.6831, 0.7941] | [0.4961, 0.8665] |
| 512 | 0.7354 | [0.6320, 0.7885] | [0.4446, 0.8666] |
| 1024 | 0.7171 | [0.6276, 0.7780] | [0.3611, 0.8518] |

When Ryder's discrimination score median ($R_i$) is below 0.7, corresponding approximately to the concentration range below 16-64 pM or so, $R_i$ increases when the concentration of the gene increases. Beyond this range, $R_i$ may decrease as gene concentration increases, but remains higher than 0.7. Therefore, $R_i$ is not always an increasing function of gene concentration, especially when median($R_i$) is above 0.7, but for the purpose of absolute calls, i.e., to detect the existence of a gene above a small concentration, e.g., 1 or 2 pM, Ryder's discrimination score $R_i$ is a very good statistic to use.

Saturation is the situation when the brightness of the pixels exceeds the brightness range of the scanner. When there is saturation $PM_i$ and $MM_i$ can be both large and close to each other, thus Ryder's discrimination score becomes small. To prevent this situation from interfering our analysis, a first check may be necessary to determine whether there are any saturated cells, and exclude them from further analysis. If all probe pairs are saturated, the gene may be indicated as detected, i.e., set the $p$-value to be 0.

Table 2.

| Statistics of $R_i$ by Concentration in pMol For a Yeast Latin Square Data Set 9912072 with Human Genome Background | | | |
|---|---|---|---|
| Concentration | Median | Interquartile range | Minimum-maximum range |
| 0 | 0.0065 | [-0.0058, 0.0225] | [-0.046, 0.47] |
| 0.25 | 0.0366 | [0.0 94, 0.0604] | [-0.0 50, 0.2676] |
| 0.5 | 0.0589 | [0.0336, 0.073] | [-0.0082, 0.3235] |
| 1 | 0.0970 | [0.0698, 0.536] | [0.0032, 0.4557] |
| 2 | 0.1704 | [0.1159, 0.2465] | [0.0152, 0.5834] |
| 4 | 0.2638 | [0.1760, 0.3602] | [0.0275, 0.6784] |
| 8 | 0.4112 | [0.3420, 0.4947] | [0.0871, 0.6942] |
| 16 | 0.5368 | [0.3807, 0.5967] | [0.1048, 0.7424] |
| 32 | 0.6228 | [0.5469, 0.6849] | [0.37, 0.7752] |
| 64 | 0.6971 | [0.6524, 0.7537] | [0.2847, 0.8300] |
| 128 | 0.7183 | [0.6588, 0.7726] | [0.3311, 0.8615] |
| 256 | 0.7324 | [0.6313, 0.7762] | [0.4198, 0.8612] |

Table 2.   (continued)

| Statistics of $R_i$ by Concentration in pMol For a Yeast Latin Square Data Set 9912072 with Human Genome Background | | | |
|---|---|---|---|
| Concentration | Median | Interquartile range | Minimum-maximum range |
| 512 | 0.7252 | [0.6151, 0.7842] | [0.4038, 0.8505] |
| 1024 | 0.7081 | [0.6168, 0.7524] | [0.4048, 0.8317] |

*Example* 2. In a semi-blind test, 11 yeast target genes were used in a hybridization solution and the yeast genome chip YG_S98 was used. The concentration of every yeast gene was 5 pM. This was a semi-blind because the algorithm developer only knew the number of target yeast genes, but neither their names nor the number and names of bacterial spiked genes. Table 3 lists the 25 sorted *p*-values of absolute calls for $p < \alpha_1 = 0.05$ with the one-sided signed rank test (1). The parameter $\tau_1$ was obtained with Equation (3c) where $c''_1 = 1.2$. The first 23 units gave the correct answer of 13 units of 11 yeast genes (YAL038W, YDL235C, YEL003W, YEL018W, YEL024W, YER161C, YFL018C, &KL193C, YLR083C, YNL259C, YPR129W) and 9 units of four bacterial spiked genes (BioB, BioC, BioDn and CreX) with a false positive. The *p*-value, 0.001602, of the unit YEL003W_at with target in hybridization the solution has a relatively large difference with the *p*-value, 0.003906, of the 24th unit NNL069W_i_at whose target is not in the hybridization solution. Therefore, if the significance level is chosen somewhere between these two numbers, a clear cutoff can be obtained.

Table 3.

| The smallest 25 *p* -values for Absolute Calls in a Test with 11 Yeast Targets and 4 Bacterial Genes | | | | | | |
|---|---|---|---|---|---|---|
| Index | p-value | $\tau_1$ | Unit No. | Unit Name | Prob e Pairs | P/A |
| 1 | 0.000044 | 51.6 | 11 | AFFX-BioB-M_at | 20 | P |
| 2 | 0.000044 | 54.3 | 13 | AFFX-BioC-5_at | 20 | P |
| 3 | 0.000044 | 44.1 | 15 | AFFX-BioC-3_at | 20 | P |
| 4 | 0.000044 | 31.7 | 16 | AFFX-BioDn-5_at | 20 | P |
| 5 | 0.000044 | 91.3 | 18 | AFFX-BioDn-3_at | 20 | P |
| 6 | 0.000044 | 95.2 | 97 | AFFX-YEL0 8w/_at | 20 | P |
| 7 | 0.000052 | 40.3 | 10 | AFFX-BioB-5_at | 20 | P |
| 8 | 0.000052 | 70.8 | 12 | AFFX-BioB-3_at | 20 | P |
| 9 | 0.000052 | 58.8 | 21 | AFFX-CreX-3_at | 20 | P |
| 10 | 0.000070 | 46.6 | 19 | AFFX-CreX-5_at | 20 | P |
| 11 | 0.000219 | 173.3 | 6105 | YFL0 8C_at | 16 | P |
| 12 | 0.000219 | 96.5 | 5941 | YER 161C_at | 16 | P |
| 13 | 0.000219 | 123.5 | 721 | YKL 193C_at | 16 | P |
| 14 | 0.000219 | 191.9 | 131 | YAL038W_at | 16 | P |
| 15 | 0.000258 | 120.6 | 98 | AFFX-YEL024w/RIP_at | 20 | P |
| 16 | 0.000266 | 95.6 | 1243 | YLR083C_at | 16 | P |
| 17 | 0.000266 | 111.1 | 5750 | YEL0 8W_at | 16 | P |
| 18 | 0.000322 | 96.0 | 2371 | YNL259C_at | 16 | P |
| 19 | 0.000388 | 34.9 | 4773 | YDL235C_at | 16 | P |
| 20 | 0.000388 | 196.9 | 5744 | YEL024W_at | 16 | P |
| 21 | 0.000468 | 81.7 | 3873 | YPR129W_at | 16 | P |
| 22 | 0.001141 | 13.7 | 3874 | YPR130C_at | 16 | A |

Table 3. (continued)

| The smallest 25 $p$ -values for Absolute Calls in a Test with 11 Yeast Targets and 4 Bacterial Genes | | | | | | |
|---|---|---|---|---|---|---|
| Index | p-value | $\tau_1$ | Unit No. | Unit Name | Prob e Pairs | P/A |
| 23 | 0.001602 | 57.6 | 5766 | YEL003W_at | 16 | P |
| 24 | 0.003906 | 11.8 | 2722 | NNL069W_i_at | 9 | A |
| 25 | 0.028446 | 11.4 | 28 | AFFX-BioDn-3_st | 20 | A |

In Table 3, the column labeled Probe Pairs lists the number of probe pairs in the unit. The column labeled P/A denotes whether the corresponding target is present (P) or absent (A) in the hybridization solution.

With Ryder's discrimination score and t2 = 0.02, sorting the p-values in the ascending order, similar results were obtained and shown in Table 4 and $p$-values 0.009985 for YEL003W_at in the hybridization solution and 0.023438 of gMR07_3_at not in the hybridization solution have a big gap.

Table 4.

| The smallest 25 p-values for Absolute Calls inaTest with Yeast Targets and 4 Bacterial Genes Using Ryder's Discrimination Score | | | | | |
|---|---|---|---|---|---|
| Index | p-value | Unit No | Unit Name | Probe Pairs | P/A |
| 1 | 0.000044 | 10 | AFFX-BioB-5_at | 20 | P |
| 2 | 0.000044 | 12 | AFFX-BioB-3_at | 20 | P |
| 3 | 0.000044 | 13 | AFFX-BioC-5_at | 20 | P |
| 4 | 0.000044 | 15 | AFFX-BioC-3_at | 20 | P |
| 5 | 0.000044 | 18 | AFFX-BioDn-3_at | 20 | P |
| 6 | 0.000044 | 19 | AFFX-CreX-5_at | 20 | P |
| 7 | 0.000052 | 11 | AFFX-BioB-M_at | 20 | P |
| 8 | 0.000052 | 21 | AFFX-CreX-3_at | 20 | P |
| 9 | 0.000052 | 97 | AFFX-YEL0 8w/_at | 20 | P |
| 10 | 0.000060 | 16 | AFFX-BioDn-5_at | 20 | P |
| 11 | 0.000219 | 1243 | YLR083C_at | 16 | P |
| 12 | 0.000219 | 5750 | YEL0 8W_at | 16 | P |
| 13 | 0.000219 | 5941 | YER161C_at | 16 | P |
| 14 | 0.000219 | 6105 | YFL018C_at | 16 | P |
| 15 | 0.000266 | 131 | YAL038W_at | 16 | P |
| 16 | 0.000390 | 98 | AFFX-YEL024w/RIP at | 20 | P |
| 17 | 0.000468 | 721 | YKL193C_at | 16 | P |
| 18 | 0.000673 | 3873 | YPR129W_at | 16 | P |
| 19 | 0.000805 | 5744 | YEL024W_at | 16 | P |
| 20 | 0.001892 | 4773 | YDL235C_at | 16 | P |
| 21 | 0.002617 | 3874 | YPR130C_at | 16 | A |
| 22 | 0.002930 | 2722 | NNL069W_i_at | 9 | A |
| 23 | 0.006532 | 2371 | YNL259C_at | 16 | P |
| 24 | 0.009985 | 5766 | YEL003W_at | 16 | P |
| 25 | 0.023438 | 8862 | gMR07_3_at | 8 | A |

*Example 3*. In a Latin square experiment design, 14 groups of yeast gene transcripts (8 genes per group) with different concentrations were used in 14 experiments (Table 5). In some of the data sets, human genome background was also added in the hybridization solution. These hybridization solutions were used on yeast genome chips yg_s95 and yeast test chips test_hyb.

calculating a *p* value using one sided Wilcoxon's signed rank test, wherein said *p* value is for a null hypothesis that $\theta$=a threshold value and an alternative hypothesis that said $\theta$> said threshold value, wherein said $\theta$ is a test statistic for intensity difference between said perfect match intensity values and background intensity values; and

indicating whether said transcript is present based upon said *p* value. the other with human genome background. If lower false positive rate is wanted, one can lower $\tau_2$, or raise $\alpha_1$ and/or $\alpha_2$

Table 6.

| Error Rates of Absolute Calls for Data Set 99802 | | | |
|---|---|---|---|
| Concentration | Marginal as undetected | Marginal as detected | Type of error |
| 0 | 0.0562 | 0.0798 | false positive |
| 0.25 | 0.0991 | 0.0811 | false negative |
| 0.5 | 0.0270 | 0.0180 | false negative |
| 1 | 0.0270 | 0.0090 | false negative |
| 2 | 0 | 0 | false negative |
| 4 | 0 | 0 | false negative |
| 8 | 0 | 0 | false negative |
| 16 | 0 | 0 | false negative |
| 32 | 0 | 0 | false negative |
| 64 | 0 | 0 | false negative |
| 128 | 0 | 0 | false negative |
| 256 | 0 | 0 | false negative |
| 512 | 0 | 0 | false negative |
| 1024 | 0 | 0 | false negative |

The column labeled by "Marginal as undetected" shows the error rates when the marginal calls are counted as undetected calls, i.e., $\alpha_1$ is used as the cutoff. The column labeled by "Marginal as detected" shows the error rates when the marginal calls are counted as detected calls, i.e., $\alpha_2$ is used as the cutoff.

Table 7.

| Error Rates of Absolute Calls for Data Set 99802BG | | | |
|---|---|---|---|
| Concentration | Marginal as undetected | Marginal as detected | Type of error |
|  |  |  |  |
| 0 | 0.0388 | 0.0524 | false positive |
| 0.25 | 0.7027 | 0.6937 | false negative |
| 0.5 | 0.5586 | 0.5135 | false negative |
| 1 | 0.3063 | 0.2883 | false negative |
| 2 | 0.0991 | 0.0811 | false negative |
| 4 | 0.0360 | 0.0180 | false negative |
| 8 | 0 | 0 | false negative |
| 16 | 0 | 0 | false negative |
| 32 | 0 | 0 | false negative |

Table 7. (continued)

| Error Rates of Absolute Calls for Data Set 99802BG | | | |
|---|---|---|---|
| Concentration | Marginal as undetected | Marginal as detected | Type of error |
| 64 | 0 | 0 | false negative |
| 128 | 0 | 0 | false negative |
| 256 | 0 | 0 | false negative |
| 512 | 0 | 0 | false negative |
| 1024 | 0 | 0 | false negative |

**Conclusion**

The present inventions provide methods and systems for analyzing gene expression profiles. It is to be understood that the above description is intended to be illustrative and not restrictive. Many variations of the invention will be apparent to those of skill in the art upon reviewing the above description. By way of example, the invention has been described primarily with reference to the use of a high density oligonucleotide array, but it will be readily recognized by those of skill in the art that other nucleic acid arrays, other methods of measuring transcript levels and gene expression monitoring at the protein level could be used. The scope of the invention should, therefore, be determined not with reference to the above description, but should instead be determined with reference to the appended claims, along with the full scope of equivalents to which such claims are entitled.

**Claims**

1. A method for determining whether a transcript is present in a biological sample comprising:

   providing a probe array hybridized to transcripts from the biological sample or nucleic acids derived therefrom;

   determining a plurality of perfect match intensity values (PMi) and mismatch intensity values (MMi) for at least one of the transcripts from hybridization intensities of the transcript to probes in the array, wherein each of the PMi is paired with one of the MMi;

   calculating a p-value using one-sided Wilcoxon's signed rank test, wherein the p-value is for a null hypothesis that $\theta$= a threshold value and an alternative hypothesis that said $\theta$> said threshold value, wherein said $\theta$ is a test statistic for intensity difference between said perfect match intensity values and mismatch intensity values; and

   indicating whether said transcript is present based upon said p-value.

2. The method of Claim 1 wherein said testing statistic is $median(PM_i\text{-}MM_i)$.

3. The method of Claim 2 wherein said threshold value is zero.

4. The method of Claim 2 wherein said threshold value is calculated using: $\tau_1 = c_1\sqrt{median(PM_i)}$ wherein said $c_1$ is a constant.

5. The method of Claim 2 wherein threshold value is calculated using: $\tau_1 = c_1\sqrt{mean(PM_i)}$ wherein said $c_1$ is a constant.

6. The method of Claim 2 wherein said step of indicating comprises indicating said transcript is present if said $p$ is smaller than a first significance level ($\alpha_1$).

7. The method of Claim 6 wherein said significance level is 0.01-0.08.

8. The method of Claim 7 wherein said first significance level is 0.04.

9. The method of Claim 7 wherein said step of indicating further comprises indicating said transcript is absent if said $p$ is greater than or equal to a second significance level ($\alpha_2$).

10. The method of Claim 9 wherein said second significance level is 0.04-0.08.

11. The method of Claim 10 wherein said second significance level is 0.06.

12. The method of Claim 11 wherein said first significance level ($\alpha_1$) is smaller than said ($\alpha_2$) and said step of indicating further comprises indicating said transcript is marginally detected if $\alpha_1 \le p < \alpha_2$.

13. The method of Claim 12 where first significance level is 0.04 and second significance level is 0.06.

14. The method of Claim 1 wherein said testing statistic is *median((PM_i- MM_i)/(PM+MM_i))*.

15. The method of Claim 14 wherein said threshold value is a constant.

16. The method of Claim 15 wherein said threshold value is around 0.001 to 0.05.

17. The method of Claim 16 wherein said threshold value is around 0.015.

18. The method of Claim 17 wherein said step of indicating comprises indicating said transcript is present if said $p$ is smaller than a first significance level ($\alpha_1$).

19. The method of Claim 18 wherein said significance level is 0.01-0.08.

20. The method of Claim 19 wherein said first significance level is 0.04.

21. The method of Claim 20 wherein said step of indicating further comprises indicating said transcript is absent if said $p$ is greater than a second significance level ($\alpha_2$).

22. The method of Claim 21 wherein said second significance level is 0.04-0.08.

23. The method of Claim 22 wherein said second significance level is 0.06.

24. The method of Claim 22 wherein said first significance level ($\alpha_1$) is sma said ($\alpha_2$) and said step of indicating further comprises indicating said transcript marginally detected if $\alpha_1 \le p < \alpha_2$.

25. The method of Claim 24 where first significance level is 0.04 and second significance level is 0.06.

26. A method for determining whether a transcript is present in a biological comprising:

   providing a probe array hybridized to transcripts from the biological sample or nucleic acids derived therefrom;
   providing a plurality of perfect match intensity values ($PM_i$) and background intensity values ($B_i$): for at least one of the transcripts from hybridization intensities of the transcript to probes in the array, wherein each of said $PM_i$ is paired with one of said $B_i$;
   calculating a $p$ value using one sided Wilcoxon's signed rank test, wherein said $p$ value is for a null hypothesis that $\theta$=a threshold value and an alternative hypothesis that said $\theta$> said threshold value, wherein said $\theta$ is a test statistic for intensity difference between said perfect match intensity values and background intensity values; and
   indicating whether said transcript is present based upon said $p$ value.

27. The method of Claim 26 wherein said testing statistic is *median(PM_i-B_i)*.

28. The method of Claim 27 wherein said threshold value is zero.

29. The method of Claim 27 wherein said threshold value is calculated using: $\tau_1 = c_1\sqrt{median(PM_i)}$ wherein said $c_1$ is a constant.

**30.** The method of Claim 27 wherein threshold value is calculated using: $\tau_3 = c_3 \sqrt{mean(PM_i)}$ wherein said $c_3$ is a constant.

**31.** The method of Claim 27 wherein said step of indicating comprises indicating said transcript is present if said p is smaller than a first significance level ($\alpha_1$).

**32.** The method of Claim 31 wherein said significance level is 0.01-0.08.

**33.** The method of Claim 32 wherein said first significance level is 0.04.

**34.** The method of Claim 31 wherein said step of indicating further comprises indicating said transcript is absent if said p is greater than a second significance level ($\alpha_2$).

**35.** The method of Claim 34 wherein said second significance level is.0.04-0.08.

**36.** The method of Claim 35 wherein said second significance level is 0.06.

**37.** The method of Claim 35 wherein said first significance level ($\alpha_1$) is smaller than said ($\alpha_2$) and said step of indicating further comprises indicating said transcript is marginally detected if $\alpha_1 \leq p < \alpha_2$.

**38.** The method of Claim 37 where first significance level is 0.04 and second significance level is 0.06.

**39.** A system for determining whether a transcript is present in a biological sample comprising:

a probe array hybridized to transcripts from the biological sample on nucleic acids derived therefrom;
a processor; and
a memory being coupled to the processor, the memory storing a plurality of machine instructions that cause the processorto perform a plurality of logical steps when implemented by the processor, said logical step comprising:

providing a plurality of perfect match intensity values ($PM_i$) and mismatch intensity values ($MM_i$) for the transcript, wherein each of the $PM_i$ is paired with one of the $MM_i$;
calculating a $p$-value using one-sided Wilcoxon's signed rank test, wherein the $p$-value is for a null hypothesis that $\theta$=a threshold value and an alternative hypothesis that said $\theta$> said threshold value, wherein said $\theta$ is a test statistic for intensity difference between said perfect match intensity values and mismatch intensity values; and

indicating whether said transcript is present based upon said $p$-value.

**40.** The system of Claim 39 wherein said testing statistic is *median(PM_i-MM_i)*

**41.** The system of Claim 40 wherein said threshold value is zero.

**42.** The system of Claim 40 wherein said threshold value is calculated using: $\tau_1 = c_1 \sqrt{median(PM_i)}$ wherein said $c_1$ is a constant.

**43.** The system of Claim 40 wherein threshold value is calculated using: $\tau_1 = c_1 \sqrt{mean(PM_i)}$ wherein said $c_1$ is a constant.

**44.** The system of Claim 40 wherein said step of indicating comprises indicating said transcript is present if said $p$ is smaller than a first significance level ($\alpha_1$).

**45.** The system of Claim 44 wherein said significance level is 0.01-0.08.

**46.** The system of Claim 45 wherein said first significance level is 0.04.

**47.** The system of Claim 45 wherein said step of indicating further comprises indicating said transcript is absent if said $p$ is greater than or equal to a second significance level ($\alpha_2$).

**48.** The system of Claim 47 wherein said second significance level is 0.04-0.08.

**49.** The system of Claim 48 wherein said second significance level is 0.06.

**50.** The system of Claim 49 wherein said first significance level ($\alpha_1$) is smaller than said ($\alpha_2$) and said step of indicating further comprises indicating said transcript is marginally detected if $\alpha_1 \leq p < \alpha_2$.

**51.** The system of Claim 50 where first significance level is 0.04 and second significance level is 0.06.

**52.** The system of Claim 39 wherein said testing statistic is $median((PM_i- MM_i)/(PM+MM_i))$.

**53.** The system of Claim 39 wherein said threshold value is a constant.

**54.** The system of Claim 53 wherein said threshold value is around 0.001 to 0.05.

**55.** The system of Claim 54 wherein said threshold value is around 0.015.

**56.** The system of Claim 53 wherein said step of indicating comprises indicating said transcript is present if said $p$ is smaller than a first significance level ($\alpha_1$).

**57.** The system of Claim 56 wherein said significance level is 0.01-0.08.

**58.** The system of Claim 57 wherein said first significance level is 0.04.

**59.** The system of Claim 58 wherein said step of indicating further comprises indicating said transcript is absent if said p is greater than a second significance level ($\alpha_2$).

**60.** The system of Claim 59 wherein said second significance level is 0.04-0.08.

**61.** The system of Claim 60 wherein said second significance level is 0.06.

**62.** The system of Claim 60 wherein said first significance level ($\alpha_1$) is smaller than said ($\alpha_2$) and said step of indicating further comprises indicating said transcript is marginally detected if $\alpha_1 \leq p < \alpha_2$.

**63.** The system of Claim 62 where first significance level is 0.04 and second significance level is 0.06.

**64.** A system for determining whether a transcript is present in a biological sample comprising:

a probe array by hybridized to transcripts from the biological sample or nucleic acids derived therefrom;
a processor; and
a memory being coupled to the processor, the memory storing a plurality machine instructions that cause the processor to perform a plurality of logical steps when implemented by the processor; said logical step comprising:

providing a plurality of perfect match intensity values ($PM_i$) and background intensity values ($B_i$) for said transcript, wherein each of said $PM_i$ is paired with one of said $B_i$;
calculating ap value using one sided Wilcoxon's signed rank test, wherein said $p$ value is for a null hypothesis that $\theta$=a threshold value and an alternative hypothesis that said $\theta$> said threshold value, wherein said $\theta$ is a test statistic for intensity difference between said perfect match intensity values and background intensity values; and
indicating whether said transcript is present based upon said $p$ value.

**Patentansprüche**

**1.** Verfahren zur Bestimmung, ob ein Transkript in einer biologischen Probe vorhanden ist, Schritte umfassend, bei denen man:

einen Sonden-Array bereitstellt, an das Transkripte der biologischen Probe oder Nukleinsäuren, die davon abgeleitet wurden, hybridisiert sind;

eine Vielzahl von Intensitätswerten perfekter Paarung ($PM_i$) und Intensitätswerten falscher Paarung ($MM_i$) aus den Hybridisierungsintensitäten des Transkripts an Sonden auf dem Array für mindestens ein Transkript bestimmt, wobei jeder der $PM_i$ mit einem der $MM_i$ gepaart wird;

einen $p$-Wert durch Verwendung eines einseitigen Wilcoxon Vorzeichen-Rang-Tests ("Wilcoxon's signed rank test") berechnet, wobei der $p$-Wert für eine Nullhypothese, gemäß der $\theta$ = einem Schwellenwert, und für eine alternative Hypothese, gemäß der $\theta >$ dem Schwellenwert, steht, wobei das $\theta$ eine Teststatistik für den Intensitätsunterschied zwischen den Intensitätswerten perfekter Paarung und den Intensitätswerten falscher Paarung ist; und

basierend auf dem $p$-Wert einen Hinweis auf die Gegenwart des Transkriptes erhält.

2. Verfahren gemäß Anspruch 1, bei dem die Teststatistik $Median(PM_i - MM_i)$ ist.

3. Verfahren gemäß Anspruch 2, bei dem der Schwellenwert Null ist.

4. Verfahren gemäß Anspruch 2, bei dem der Schwellenwert unter Verwendung von: $\tau_1 = c_1\sqrt{Median(PM_i)}$ berechnet wird, worin $c_1$ eine Konstante ist.

5. Verfahren gemäß Anspruch 2, bei dem der Schwellenwert unter Verwendung von: $\tau_1 = c_1\sqrt{Mittelwert(PM_i)}$ berechnet wird, worin $c_1$ eine Konstante ist.

6. Verfahren gemäß Anspruch 2, bei dem der Hinweisschritt einen Hinweis auf die Gegenwart des Transkriptes umfaßt, wenn $p$ kleiner als ein erstes Signifikanzniveau ($\alpha_1$) ist.

7. Verfahren gemäß Anspruch 6, bei dem das Signifikanzniveau 0.01-0.08 ist.

8. Verfahren gemäß Anspruch 7, bei dem das erste Signifikanzniveau 0.04 ist.

9. Verfahren gemäß Anspruch 7, bei dem der Hinweisschritt ferner den Hinweis auf die Abwesenheit des Transkriptes umfaßt, wenn $p$ größer oder gleich einem zweiten Signifikanzniveau ($\alpha_2$) ist.

10. Verfahren gemäß Anspruch 9, bei dem das zweite Signifikanzniveau 0.04-0.08 ist.

11. Verfahren gemäß Anspruch 10, bei dem das zweite Signifikanzniveau 0.06 ist.

12. Verfahren gemäß Anspruch 11, bei dem das erste Signifikanzniveau ($\alpha_1$) kleiner als ($\alpha_2$) ist, und der Hinweisschritt außerdem den Hinweis umfaßt, dass das Transkript geringfügig nachweisbar ist, wenn $\alpha_1 \leq p < \alpha_2$ ist.

13. Verfahren gemäß Anspruch 12, bei dem das erste Signifikanzniveau 0.04 und das zweite Signifikanzniveau 0.06 ist.

14. Verfahren gemäß Anspruch 1, bei dem die Teststatistik $Median((PM_i - MM_i)/(PM_+MM_i))$ ist.

15. Verfahren gemäß Anspruch 14, bei dem der Schwellenwert eine Konstante ist.

16. Verfahren gemäß Anspruch 15, bei dem der Schwellenwert um 0.001 bis 0.05 liegt.

17. Verfahren gemäß Anspruch 16, bei dem der Schwellenwert um 0.015 liegt.

18. Verfahren gemäß Anspruch 17, bei dem der Hinweisschritt einen Hinweis auf die Gegenwart des Transkriptes umfaßt, wenn $p$ kleiner als ein erstes Signifikanzniveau ($\alpha_1$) ist.

19. Verfahren gemäß Anspruch 18, bei dem das Signifikanzniveau 0.01-0.08 ist.

20. Verfahren gemäß Anspruch 19, bei dem das erste Signifikanzniveau 0.04 ist.

21. Verfahren gemäß Anspruch 20, bei dem der Hinweisschritt ferner einen Hinweis auf die Abwesenheit des Transkriptes umfaßt, wenn p größer als ein zweites Signifikanzniveau ($\alpha_2$) ist.

22. Verfahren gemäß Anspruch 21, bei dem das zweite Signifikanzniveau 0.04-0.08 ist.

23. Verfahren gemäß Anspruch 22, bei dem das zweite Signifikanzniveau 0.06 ist.

24. Verfahren gemäß Anspruch 22, bei dem das erste Signifikanzniveau ($\alpha_1$) kleiner als ($\alpha_2$) ist, und der Hinweisschritt ferner den Hinweis umfaßt, dass das Transkript geringfügig nachweisbar ist, wenn $\alpha_1 \leq p < \alpha_2$ ist.

25. Verfahren gemäß Anspruch 24, bei dem das erste Signifikanzniveau 0.04 und das zweite Signifikanzniveau 0.06 ist.

26. Verfahren zur Bestimmung, ob ein Transkript in einer biologischen Probe vorhanden ist, Schritte umfassend, bei denen man:

   einen Sonden-Array bereitstellt, an das Transkripte der biologischen Probe oder Nukleinsäuren, die davon abgeleitet wurden, hybridisiert sind;

   eine Vielzahl von Intensitätswerten perfekter Paarung ($PM_i$) und Intensitätswerten Hintergrund ($B_i$) aus den Hybridisierungsintensitäten des Transkriptes an Sonden auf dem Array für mindestens ein Transkript bereitstellt, wobei jeder der $PM_i$ mit einem der $B_i$ gepaart ist;

   einen $p$-Wert durch Verwendung eines einseitigen Wilcoxon Vorzeichen-Rang-Tests berechnet, wobei der $p$-Wert für eine Nullhypothese, gemäß der $\theta$ = einem Schwellenwert, und für eine alternative Hypothese, gemäß der $\theta >$ dem Schwellenwert, steht, wobei das $\theta$ eine Teststatistik für den Intensitätsunterschied zwischen den Intensitätswerten perfekter Paarung und den Intensitätswerten Hintergrund ist; und

   basierend auf dem $p$-Wert einen Hinweis auf die Gegenwart des Transkriptes erhält.

27. Verfahren gemäß Anspruch 26, bei dem die Teststatistik $Median(PM_i\text{-}B_i)$ ist.

28. Verfahren gemäß Anspruch 27, bei dem der Schwellenwert Null ist.

29. Verfahren gemäß Anspruch 27, bei dem der Schwellenwert unter Verwendung von: $\tau_1 = c_1\sqrt{Median(PM_i)}$ berechnet wird, worin $c_1$ eine Konstante ist.

30. Verfahren gemäß Anspruch 27, bei dem der Schwellenwert unter Verwendung von: $\tau_3 = c_3\sqrt{Mittelwert(PM_i)}$ berechnet wird, worin $c_3$ eine Konstante ist.

31. Verfahren gemäß Anspruch 27, bei dem der Hinweisschritt einen Hinweis auf die Gegenwart des Transkriptes umfaßt, wenn p kleiner als ein erstes Signifikanzniveau ($\alpha_1$) ist.

32. Verfahren gemäß Anspruch 31, bei dem das Signifikanzniveau 0.01-0.08 ist.

33. Verfahren gemäß Anspruch 32, bei dem das erste Signifikanzniveau 0.04 ist.

34. Verfahren gemäß Anspruch 31, bei dem der Hinweisschritt ferner einen Hinweis auf die Abwesenheit des Transkriptes umfaßt, wenn $p$ größer als ein zweites Signifikanzniveau ($\alpha_2$) ist.

35. Verfahren gemäß Anspruch 34, bei dem das zweite Signifikanzniveau 0.04-0.08 ist.

36. Verfahren gemäß Anspruch 35, bei dem das zweite Signifikanzniveau 0.06 ist.

37. Verfahren gemäß Anspruch 35, bei dem das erste Signifikanzniveau ($\alpha_1$) kleiner als ($\alpha_2$) ist, und der Hinweisschritt ferner einen Hinweis darauf umfaßt, dass das Transkript geringfügig nachweisbar ist, wenn $\alpha_1 \leq p < \alpha_2$ ist.

38. Verfahren gemäß Anspruch 37, bei dem das erste Signifikanzniveau 0.04 und das zweite Signifikanzniveau 0.06 ist.

**39.** System zur Bestimmung, ob ein Transkript in einer biologischen Probe vorhanden ist, umfassend:

einen Sonden-Array, an das Transkripte der biologischen Probe oder Nukleinsäuren, die davon abgeleitet wurden, hybridisiert sind;

einen Prozessor; und

einen Speicher, der mit dem Prozessor verbunden ist, wobei der Speicher eine Vielzahl von Maschinenbefehlen speichert, die den Prozessor veranlassen, eine Vielzahl logischer Schritte von dem Prozessor implementiert auszuführen, wobei der logische Schritt umfaßt:

Bereitstellung einer Vielzahl von Intensitätswerten perfekter Paarung ($PM_i$) und Intensitätswerten falscher Paarung ($MM_i$) für das Transkript, wobei jeder der $PM_i$ mit einem der $MM_i$ gepaart ist;

Berechnung eines $p$-Wertes durch Verwendung eines einseitigen Wilcoxon Vorzeichen-Rang-Tests, wobei der $p$-Wert für eine Nullhypothese, gemäß der $\theta$ = einem Schwellenwert, und für eine alternative Hypothese, gemäß der $\theta$ > dem Schwellenwert, steht, wobei das $\theta$ eine Teststatistik für den Intensitätsunterschied zwischen den Intensitätswerten perfekter Paarung und den Intensitätswerten falscher Paarung ist; und

basierend auf dem $p$-Wert Erhalt eines Hinweises auf die Gegenwart des Transkriptes.

**40.** System gemäß Anspruch 39, bei dem die Teststatistik $Median(PM_i - MM_i)$ ist.

**41.** System gemäß Anspruch 40, bei dem der Schwellenwert Null ist.

**42.** System gemäß Anspruch 40, bei dem der Schwellenwert unter Verwendung von: $\tau_1 = c_1\sqrt{Median(PM_i)}$ berechnet wird, worin $c_1$ eine Konstante ist.

**43.** System gemäß Anspruch 40, bei dem der Schwellenwert unter Verwendung von: $\tau_1 = c_1\sqrt{Mittelwert(PM_i)}$ berechnet wird, worin $c_1$ eine Konstante ist.

**44.** System gemäß Anspruch 40, bei dem der Hinweisschritt einen Hinweis auf die Gegenwart des Transkriptes umfaßt, wenn $p$ kleiner als ein erstes Signifikanzniveau ($\alpha_1$) ist.

**45.** System gemäß Anspruch 44, bei dem das Signifikanzniveau 0.01-0.08 ist.

**46.** System gemäß Anspruch 45, bei dem das erste Signifikanzniveau 0.04 ist.

**47.** System gemäß Anspruch 45, bei dem der Hinweisschritt ferner einen Hinweis auf die Abwesenheit des Transkriptes umfaßt, wenn $p$ größer oder gleich einem zweiten Signifikanzniveau ($\alpha_2$) ist.

**48.** System gemäß Anspruch 47, bei dem das zweite Signifikanzniveau 0.04-0.08 ist.

**49.** System gemäß Anspruch 48, bei dem das zweite Signifikanzniveau 0.06 ist.

**50.** System gemäß Anspruch 49, bei dem das erste Signifikanzniveau ($\alpha_1$) kleiner als ($\alpha_2$) ist, und der Hinweisschritt ferner einen Hinweis darauf umfaßt, dass das Transkript geringfügig nachweisbar ist, wenn $\alpha_1 \leq p < \alpha_2$ ist.

**51.** System gemäß Anspruch 50, bei dem das erste Signifikanzniveau 0.04 und das zweite Signifikanzniveau 0.06 ist.

**52.** System gemäß Anspruch 39, bei dem die Teststatistik $Median((PM_i\text{-}MM_i)/(PM_+MM_i))$ ist.

**53.** System gemäß Anspruch 39, bei dem der Schwellenwert eine Konstante ist.

**54.** System gemäß Anspruch 53, bei dem der Schwellenwert um 0.001 bis 0.05 liegt.

**55.** System gemäß Anspruch 54, bei dem der Schwellenwert um 0.015 liegt.

**56.** System gemäß Anspruch 53, bei dem der Hinweisschritt einen Hinweis auf die Gegenwart des Transkriptes umfaßt, wenn $p$ kleiner als ein erstes Signifikanzniveau ($\alpha_1$) ist.

**57.** System gemäß Anspruch 56, bei dem das Signifikanzniveau 0.01-0.08 ist.

**58.** System gemäß Anspruch 57, bei dem das erste Signifikanzniveau 0.04 ist.

**59.** System gemäß Anspruch 58, bei dem der Hinweisschritt ferner einen Hinweis auf die Abwesenheit des Transkriptes umfaßt, wenn $p$ größer als ein zweites Signifikanzniveau ($\alpha_2$) ist.

**60.** System gemäß Anspruch 59, bei dem das zweite Signifikanzniveau 0.04-0.08 ist.

**61.** System gemäß Anspruch 60, bei dem das zweite Signifikanzniveau 0.06 ist.

**62.** System gemäß Anspruch 60, bei dem das erste Signifikanzniveau ($\alpha_1$) kleiner als ($\alpha_2$) ist, und der Hinweisschritt ferner den Hinweis umfaßt, dass das Transkript geringfügig nachweisbar ist, wenn $\alpha_1 \leq p < \alpha_2$ ist.

**63.** System gemäß Anspruch 62, bei dem das erste Signifikanzniveau 0.04 und das zweite Signifikanzniveau 0.06 ist.

**64.** System zur Bestimmung, ob ein Transkript in einer biologischen Probe anwesend ist, umfassend:

einen Sonden-Array, an das Transkripte der biologischen Probe oder Nukleinsäuren, die davon abgeleitet wurden, hybridisiert sind;

einen Prozessor; und

einen Speicher, der mit dem Prozessor verbunden ist, wobei der Speicher eine Vielzahl von Maschinenbefehlen speichert, die den Prozessor veranlassen, eine Vielzahl logischer Schritte von dem Prozessor implementiert auszuführen, wobei der logische Schritt umfaßt:

Bereitstellung einer Vielzahl von Intensitätswerten perfekter Paarung ($PM_i$) und Intensitätswerten Hintergrund ($B_i$) für das Transkript, wobei jeder der $PM_i$ mit einem der $B_i$ gepaart ist;

Berechnung eines $p$-Wertes durch Verwendung eines einseitigen Wilcoxon Vorzeichen-Rang-Tests, wobei der $p$-Wert für eine Nullhypothese, gemäß der $\theta$ = einem Schwellenwert, und für eine alternative Hy-pothese, gemäß der $\theta$ > dem Schwellenwert, steht, wobei das $\theta$ eine Teststatistik für den Intensitätsunterschied zwischen den Intensitätswerten perfekter Paarung und den Intensitätswerten Hintergrund ist; und

basierend auf dem p-Wert Erhalt eines Hinweises auf die Gegenwart des Transkriptes.

**Revendications**

**1.** Procédé servant à déterminer si un transcrit est présent ou non dans un échantillon biologique, comprenant les étapes consistant :

à fournir une matrice de sondes hybridée à des transcrits provenant de l'échantillon biologique ou d'acides nucléiques dérivées de celui-ci ;

à déterminer une pluralité de valeurs d'intensité d'appariement parfait ($PM_i$) et de valeurs d'intensité de mésappariement ($MM_i$), pour au moins l'un des transcrits, d'intensités d'hybridation du transcrit par rapport à des sondes dans la matrice, dans lequel chacune des $PM_i$ est appariée avec l'une des $MM_i$ ;

à calculer une valeur $p$ en utilisant le test unilatéral de Wilcoxon des rangs signés, dans lequel la valeur $p$ est pour une hypothèse nulle que $\theta$ est égal à une valeur seuil et pour une hypothèse alternative que ledit $\theta$ est supérieur à ladite valeur seuil, dans lequel ledit $\theta$ est une statistique de test pour une différence d'intensité entre lesdites valeurs d'appariement parfait et lesdites valeurs d'intensité de mésappariement ; et

à indiquer si ledit transcrit et présent ou non, sur la base de ladite valeur $p$.

2. Procédé selon la revendication 1, dans lequel ladite statistique de test est *médiane($PM_i$ - $MM_i$)*.

3. Procédé selon la revendication 2, dans lequel ladite valeur seuil est de zéro.

4. Procédé selon la revendication 2, dans lequel ladite valeur seuil est calculée en utilisant la formule $\tau_1 = c_1$ *racine ( médiane($PM_i$))*, dans laquelle ledit $c_1$ est une constante.

5. Procédé selon la revendication 2, dans lequel ladite valeur seuil est calculée en utilisant la formule $\tau_1 = c_1$ *racine ( moyenne($PM_i$) )*, dans laquelle ledit $c_1$ est une constante.

6. Procédé selon la revendication 2, dans lequel ladite étape consistant à indiquer comprend le fait d'indiquer que ledit transcrit est présent, si ledit p est inférieur à un premier niveau de signification ($\alpha_1$).

7. Procédé selon la revendication 6 dans lequel ledit premier niveau de signification se situe entre 0,01 et 0,08.

8. Procédé selon la revendication 7, dans lequel ledit premier niveau de signification est de 0,04.

9. Procédé selon la revendication 7, dans lequel ladite étape consistant à indiquer comprend en outre le fait d'indiquer que ledit transcrit est absent, si ledit p est supérieur ou égal à un deuxième niveau de signification ($\alpha_2$).

10. Procédé selon la revendication 9, dans lequel ledit deuxième niveau de signification se situe entre 0,04 et 0,08.

11. Procédé selon la revendication 10, dans lequel ledit deuxième niveau de signification est de 0,06.

12. Procédé selon la revendication 11, dans lequel ledit premier niveau de signification ($\alpha_1$) est inférieur audit second niveau de signification ($\alpha_2$) et ladite étape consistant à indiquer comprend en outre le fait d'indiquer que ledit transcrit est détecté de manière marginale, si $\alpha_1 \le p < \alpha_2$.

13. Procédé selon la revendication 12, dans lequel le premier niveau de signification est de 0,04 et le deuxième niveau de signification est de 0,06.

14. Procédé selon la revendication 1, dans lequel ladite statistique de test est *médiane( ($PM_i$ - $MM_i$) / ($PM_i$ + $MM_i$) )*.

15. Procédé selon la revendication 14, dans lequel ladite valeur seuil est une constante.

16. Procédé selon la revendication 15, dans lequel ladite valeur seuil se situe entre environ 0,001 et 0,05.

17. Procédé selon la revendication 16, dans lequel ladite valeur seuil est d'environ 0,015.

18. Procédé selon la revendication 17, dans lequel ladite étape consistant à indiquer comprend le fait d'indiquer que ledit transcrit est présent, si ledit $p$ est inférieur à un premier niveau de signification ($\alpha_1$).

19. Procédé selon la revendication 18, dans lequel ledit premier niveau de signification se situe entre 0,01 et 0,08.

20. Procédé selon la revendication 19, dans lequel ledit premier niveau de signification est de 0,04.

21. Procédé selon la revendication 20, dans lequel ladite étape consistant à indiquer comprend en outre le fait d'indiquer que ledit transcrit est absent, si ledit p est supérieur à un deuxième niveau de signification ($\alpha_2$).

22. Procédé selon la revendication 21, dans lequel ledit deuxième niveau de signification se situe entre 0,04 et 0,08.

23. Procédé selon la revendication 22, dans lequel ledit deuxième niveau de signification est de 0,06.

24. Procédé selon la revendication 22, dans lequel ledit premier niveau de signification ($\alpha_1$) est inférieur audit deuxième niveau de signification ($\alpha_2$) et ladite étape consistant à indiquer comprend en outre le fait d'indiquer que ledit transcrit est détecté de manière marginale, si $\alpha_1 \le p < \alpha_2$.

**25.** Procédé selon la revendication 24, dans lequel le premier niveau de signification est de 0,04 et le deuxième niveau de signification est de 0,06.

**26.** Procédé servant à déterminer si un transcrit est présent ou non dans un échantillon biologique, comprenant les étapes consistant :

à fournir une matrice de sondes hybridée à des transcrits provenant de l'échantillon biologique ou d'acides nucléiques dérivées de celui-ci ;

à fournir une pluralité de valeurs d'intensité d'appariement parfait ($PM_i$) et de valeurs d'intensité de fond ($B_i$), pour au moins l'un des transcrits, d'intensités d'hybridation du transcrit par rapport à des sondes dans la matrice, dans lequel chacune desdites $PM_i$ est appariée avec l'une desdites $B_i$ ;

à calculer une valeur $p$ en utilisant le test unilatéral de Wilcoxon des rangs signés, dans lequel la valeur $p$ est pour une hypothèse nulle que $\theta$ est égal à une valeur seuil et pour une hypothèse alternative que ledit $\theta$ est supérieur à ladite valeur seuil, dans lequel ledit $\theta$ est une statistique de test pour une différence d'intensité entre lesdites valeurs d'appariement parfait et lesdites valeurs d'intensité de mésappariement ; et

à indiquer si ledit transcrit et présent ou non, sur la base de la dite valeur $p$.

**27.** Procédé selon la revendication 26, dans lequel ladite statistique de test est *médiane($PM_i$ - $B_i$)*.

**28.** Procédé selon la revendication 27, dans lequel ladite valeur seuil est de zéro.

**29.** Procédé selon la revendication 27, dans lequel ladite valeur seuil est calculée en utilisant la formule $\tau_1 = c_1$ *racine (médiane($PM_i$)),* dans laquelle ledit $c_1$ est une constante.

**30.** Procédé selon la revendication 27, dans lequel ladite valeur seuil est calculée en utilisant la formule $\tau_3 = c_3$ *racine ( moyenne($PM_i$) ),* dans laquelle ledit $c_3$ est une constante.

**31.** Procédé selon la revendication 27, dans lequel ladite étape consistant à indiquer comprend le fait d'indiquer que ledit transcrit est présent, si ledit p est inférieur à un premier niveau de signification ($\alpha_1$).

**32.** Procédé selon la revendication 31, dans lequel ledit premier niveau de signification se situe entre 0,01 et 0,08.

**33.** Procédé selon la revendication 32, dans lequel ledit premier niveau de signification est de 0,04.

**34.** Procédé selon la revendication 31, dans lequel ladite étape consistant à indiquer comprend en outre le fait d'indiquer que ledit transcrit est absent, si ledit $p$ est supérieur à un deuxième niveau de signification ($\alpha_2$).

**35.** Procédé selon la revendication 34, dans lequel ledit deuxième niveau de signification se situe entre 0,04 et 0,08.

**36.** Procédé selon la revendication 35, dans lequel ledit deuxième niveau de signification est de 0,06.

**37.** Procédé selon la revendication 35, dans lequel ledit premier niveau de signification ($\alpha_1$) est inférieur audit deuxième niveau de signification ($\alpha_2$) et ladite étape consistant à indiquer comprend en outre le fait d'indiquer que ledit transcrit est détecté de manière marginale, si $\alpha_1 \leq p < \alpha_2$.

**38.** Procédé selon la revendication 37, dans lequel le premier niveau de signification est de 0,04 et le deuxième niveau de signification est de 0,06.

**39.** Système servant à déterminer si un transcrit est présent ou non dans un échantillon biologique, comprenant :

une matrice de sondes hybridée à des transcrits provenant de l'échantillon biologique ou d'acides nucléiques dérivées de celui-ci ;

un processeur ; et

une mémoire étant couplée au processeur, la mémoire stockant une pluralité d'instructions machine qui font en sorte que le processeur exécute une pluralité d'étapes logiques lorsque implémentées par le processeur, lesdites étapes logiques comprenant les étapes consistant :

à fournir une pluralité de valeurs d'intensité d'appariement parfait ($PM_i$) et de valeurs d'intensité de mésappariement ($MM_i$) pour le transcrit, dans lequel chacune des $PM_i$ est appariée avec l'une des $MM_i$ ;

à calculer une valeur $p$ en utilisant le test unilatéral de Wilcoxon des rangs signés, dans lequel la valeur $p$ est pour une hypothèse nulle que $\theta$ est égal à une valeur seuil et pour une hypothèse alternative que ledit $\theta$ est supérieur à ladite valeur seuil, dans lequel ledit $\theta$ est une statistique de test pour une différence d'intensité entre lesdites valeurs d'appariement parfait et lesdites valeurs d'intensité de mésappariement ; et

à indiquer si ledit transcrit et présent ou non, sur la base de la dite valeur $p$.

40. Système selon la revendication 39, dans lequel ladite statistique de test est *médiane($PM_i$ - $MM_i$)*.

41. Système selon la revendication 40, dans lequel ladite valeur seuil est de zéro.

42. Système selon la revendication 40, dans lequel ladite valeur seuil est calculée en utilisant la formule $\tau_1 = c_1$ *racine ( médiane($PM_i$) )*, dans laquelle ledit $c_1$ est une constante.

43. Système selon la revendication 40, dans lequel ladite valeur seuil est calculée en utilisant la formule $\tau_1 = c_1$ *racine ( moyenne($PM_i$) )*, dans laquelle ledit $c_1$ est une constante.

44. Système selon la revendication 40, dans lequel ladite étape consistant à indiquer comprend le fait d'indiquer que ledit transcrit est présent, si ledit $p$ est inférieur à un premier niveau de signification ($\alpha_1$).

45. Système selon la revendication 44 dans lequel ledit premier niveau de signification se situe entre 0,01 et 0,08.

46. Système selon la revendication 45, dans lequel ledit premier niveau de signification est de 0,04.

47. Système selon la revendication 45, dans lequel ladite étape consistant à indiquer comprend en outre le fait d'indiquer que ledit transcrit est absent, si ledit p est supérieur ou égal à un deuxième niveau de signification ($\alpha_2$).

48. Système selon la revendication 47, dans lequel ledit deuxième niveau de signification se situe entre 0,04 et 0,08.

49. Système selon la revendication 48, dans lequel ledit deuxième niveau de signification est de 0,06.

50. Système selon la revendication 49, dans lequel ledit premier niveau de signification ($\alpha_1$) est inférieur audit deuxième niveau de signification ($\alpha_2$) et ladite étape consistant à indiquer comprend en outre le fait d'indiquer que ledit transcrit est détecté de manière marginale, si $\alpha_1 \leq p < \alpha_2$.

51. Système selon la revendication 50, dans lequel le premier niveau de signification est de 0,04 et le deuxième niveau de signification est de 0,06.

52. Système selon la revendication 39, dans lequel ladite statistique de test est *médiane( ($PM_i$- $MM_i$) / ($PM_i$ + $MM_i$) )*.

53. Système selon la revendication 39, dans lequel ladite valeur seuil est une constante.

54. Système selon la revendication 53, dans lequel ladite valeur seuil se situe entre environ 0,001 et 0,05.

55. Système selon la revendication 54, dans lequel ladite valeur seuil est d'environ 0,015.

56. Système selon la revendication 53, dans lequel ladite étape consistant à indiquer comprend le fait d'indiquer que ledit transcrit est présent, si ledit $p$ est inférieur à un premier niveau de signification ($\alpha_1$).

57. Système selon la revendication 56, dans lequel ledit premier niveau de signification se situe entre 0,01 et 0,08.

**58.** Système selon la revendication 57, dans lequel ledit premier niveau de signification est de 0,04.

**59.** Système selon la revendication 58, dans lequel ladite étape consistant à indiquer comprend en outre le fait d'indiquer que ledit transcrit est absent, si ledit p est supérieur à un deuxième niveau de signification ($\alpha_2$).

**60.** Système selon la revendication 59, dans lequel ledit deuxième niveau de signification se situe entre 0,04 et 0,08.

**61.** Système selon la revendication 60, dans lequel ledit deuxième niveau de signification est de 0,06.

**62.** Système selon la revendication 60, dans lequel ledit premier niveau de signification ($\alpha_1$) est inférieur audit deuxième niveau de significations ($\alpha_2$) et ladite étape consistant à indiquer comprend en outre le fait d'indiquer que ledit transcrit est détecté de manière marginale, si $\alpha_1 \leq p < \alpha_2$.

**63.** Système selon la revendication 62, dans lequel le premier niveau de signification est de 0,04 et le deuxième niveau de signification est de 0,06.

**64.** Système servant à déterminer si un transcrit est présent ou non dans un échantillon biologique, comprenant :

une matrice de sondes hybridée à des transcrits provenant de l'échantillon biologique ou d'acides nucléiques dérivées de celui-ci ;

un processeur ; et

une mémoire étant couplée au processeur, la mémoire stockant une pluralité d'instructions machine qui font en sorte que le processeur exécute une pluralité d'étapes logiques lorsque implémentées par le processeur, lesdites étapes logiques comprenant les étapes consistant:

à fournir une pluralité de valeurs d'intensité d'appariement parfait ($PM_i$) et de valeurs d'intensité de fond ($B_i$) pour ledit transcrit, dans lequel chacune desdites $PM_i$ est appariée avec l'une desdites $B_i$ ;

à calculer une valeur $p$ en utilisant le test unilatéral de Wilcoxon des rangs signés, dans lequel la valeur $p$ est pour une hypothèse nulle que $\theta$ est égal à une valeur seuil et pour une hypothèse alternative que ledit $\theta$ est supérieur à ladite valeur seuil, dans lequel ledit $\theta$ est une statistique de test pour une différence d'intensité entre lesdites valeurs d'appariement parfait et lesdites valeurs d'intensité de fond ; et

à indiquer si ledit transcrit et présent ou non, sur la base de la dite valeur $p$.

**Figure 1**

**Figure 2**

Figure 3

Input $PM_i$
$MM_i$

**41**

Calculate One-
sided $p$-value
for $H_o : \theta = 0$
$H_1 : \theta > 0$

**42**

**43**

$p < \alpha$?

No

Yes

**44**

The Transcript
is Detected

**45**

The Transcript
is Not Detected

**Figure 4**

Read Data Files — 51

Calculate Threshold
$\tau$ — 52

Calculate one-sided
p-value

$median(PM_i - MM_i) > \tau$ — 53

54

$p < \alpha_1$  —Yes→ Detected — 55

No

56

$p < \alpha_2$  —Yes→ Marginal — 57

No

Undetected — 58

Figure 5

Read
Data
File  — 61

Calculate one - sided
$p$ - value

$H_o : median \dfrac{(PM_i - MM_i)}{(PM_i + MM_i)} = \tau$

$H_1$

$H_o : median \dfrac{(PM_i - MM_i)}{(PM_i + MM_i)} > \tau$

$\tau$ is a constant

— 62

63

$p < \alpha_1$ —— Yes ——→ Detected — 64

$p < \alpha_2$ —— Yes ——→ Marginal — 66

65

Undetected — 67

Figure 6

(Amended)

Read Data File — 71

Calculate $B_i$ — 72

Calculate $\tau$ — 73

Calculate p - value — 74
$H_o : median\,(PM_i - B_i) = \tau$
$H_1 : median\,(PM_i - B_i) > \tau$

75 $P < \alpha_1$

Yes → Detected — 76

No

77 $P < \alpha_2$

Yes → Marginal — 78

No

Undetected — 79

Figure 7

Figure 8